(19) Europäisches Patentamt
European Patent Office
Office européen des brevets

(11) Veröffentlichungsnummer: **0 091 398**
**B1**

(12) **EUROPÄISCHE PATENTSCHRIFT**

(45) Veröffentlichungstag der Patentschrift: **02.09.87**

(21) Anmeldenummer: **83810082.4**

(22) Anmeldetag: **28.02.83**

(51) Int. Cl.[4]: **C 07 D 249/08,** C 07 D 233/60, A 01 N 43/653, A 01 N 43/50, C 07 D 405/06

(54) **Mikrobizide und wuchsregulierende Azolylpropan-Derivate.**

(30) Priorität: **04.03.82 CH 1329/82**
**24.11.82 CH 6850/82**

(43) Veröffentlichungstag der Anmeldung: **12.10.83 Patentblatt 83/41**

(45) Bekanntmachung des Hinweises auf die Patenterteilung: **02.09.87 Patentblatt 87/36**

(84) Benannte Vertragsstaaten: **AT BE CH DE FR GB IT LI NL SE**

(56) Entgegenhaltungen:
**EP-A-0 040 345**
**EP-A-0 043 419**
**EP-A-0 054 974**
**EP-A-0 057 357**
**EP-A-0 061 835**
**EP-A-0 070 798**
**EP-A-0 072 580**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Müller, Urs, Dr., Schluchtstrasse 15, CH- 4142 Münchenstein (CH)**
Erfinder: **Tobler, Hans, Dr., Baselmattweg 157, CH- 4123 Allschwil (CH)**
Erfinder: **Rempfler, Hermann, Dr., Brücklismattstrasse 16, CH- 4107 Ettingen (CH)**
Erfinder: **Meyer, Alfred, Dr., Bristenweg 6, CH- 4054 Basel (CH)**

EP 0 091 398 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft neue, substituierte Azolylpropan-Derivate sowie deren Säureadditionssalze, quaternäre Azoliumsalze und Metallkomplexe. Die Erfindung betrifft ferner die Herstellung dieser Substanzen sowie mikrobizide und wuchsregulierende Mittel, die als Wirkstoff mindestens eine dieser Verbindungen enthalten. Die Erfindung betrifft auch die Herstellung der genannten Mittel und die Verwendung der Wirkstoffe oder der Mittel zur Regulierung des Pflanzenwachstums und zur Bekämpfung von schädlichen Mikroorganismen. Weiter betrifft die Erfindung als Zwischenprodukte hergestellte Azolylmethyloxirane.

Es werden hierin Azolylpropan-Derivate der allgemeinen Formel I

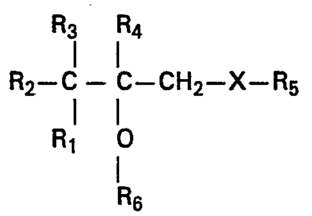

(I)

umfasst, worin

$R_1$ für 1H-1,2,4-Triazol-1-yl oder 1H-Imidazol-1-yl steht,

$R_2$ $C_1$-$C_4$-Alkyl und $R_3$ Wasserstoff, $C_1$-$C_4$-Alkyl oder gegebenenfalls einfach durch Halogen substituiertes Benzyl bedeuten,

$R_4$ für $C_1$-$C_6$-Alkyl oder für gegebenenfalls einfach durch Halogen, Cyan, Nitro, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy oder $C_1$-$C_4$-Halogenalkylthio substituiertes Phenyl steht,

$R_5$ für $C_1$-$C_8$-Alkyl, $C_3$-$C_8$-Cycloalkyl, Phenylalkyl mit 1-4 Kohlenstoffatomen im Alkylteil oder gegebenenfalls ein- oder mehrfach, durch Halogen, Cyan, Nitro, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy oder $C_1$-$C_4$-Halogenalkylthio substituiertes Phenyl steht,

$R_6$ Wasserstoff, $C_1$-$C_4$-Alkyl, Phenylalkyl mit 1-4 Kohlenstoffatomen im Alkylteil, $C_1$-$C_6$-Alkylcarbonyl, Carbamoyl, an dessen Stickstoffatom unabhängig von einander Wasserstoff, $C_1$-$C_4$-Alkoxy oder $C_1$-$C_4$-Alkyl gebunden sind, $C_1$-$C_4$-Alkoxycarbonyl, $C_1$-$C_4$-Alkylsulfonyl, gegebenenfalls einfach durch $C_1$-$C_4$-Alkyl substituiertes Phenylsulfonyl oder Sulfamoyl, an dessen Stickstoffatom unabhängig von einander Wasserstoff oder $C_1$-$C_4$-Alkyl gebunden sind, und

X Sauerstoff oder Schwefel bedeuten, unter Einschluss der Säureadditionssalze, der quaternären Azoliumsalze und der Metallkomplexe.

Unter dem Begriff Alkyl selbst oder als Bestandteil eines anderen Substituenten, wie Alkoxy, Alkylthio, Halogenalkyl, Halogenalkylthio, Aralkyl, Alkylcarbonyl, Alkylsulfonyl oder Alkylimino sind je nach Zahl der angegebenen Kohlenstoffatome folgende Gruppen zu verstehen: Methyl, Äthyl, Propyl, Butyl, Pentyl, Hexyl, Heptyl oder Octyl sowie ihre Isomeren, wie Isopropyl, Isobutyl, sec.-Butyl, tert.-Butyl, Isopentyl Halogenalkyl steht für einen einfach bis perhalogenierten Alkylsubstituenten, wie $CHCl_2$, $CHF_2$, $CH_2Cl$, $CCl_3$, $CH_2F$, $-CH_2CH_2Cl$, $CHBr_2$. Unter Halogen soll hier und im folgenden Fluor, Chlor, Brom oder Jod, vorzugsweise Fluor, Chlor oder Brom, verstanden werden. Alkenyl bedeutet Propenyl-(1), Allyl, Butenyl-(1), Butenyl-(2) oder Butenyl-(3), Alkinyl steht für Propionyl-(1) oder Propargyl. Cycloalkyl bedeutet je nach Zahl der Kohlenstoffatome Cyclopropyl, Cyclobutyl, Cyclopentyl, Cyclohexyl, Cycloheptyl, Cyclooctyl.

Die vorliegende Erfindung betrifft sowohl die freien organischen Moleküle der Formel I, als auch deren Säureadditionssalze, quaternären Azoliumsalze und Metallkomplexe. Die freien Moleküle sind bevorzugt. Salzbildende Säuren sind anorganische Säuren: Fluorwasserstoffsäure, Chlorwasserstoffsäure, Bromwasserstoffsäure, Jodwasserstoffsäure, Schwefelsäure, Phosphorsäure, phosphorige Säure, Salpetersäure und organische Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Trifluormethansulfonsäure, Salicylsäure, p-Aminosalicylsäure, 2-Phenoxybenzoesäure oder 2-Acetoxybenzoesäure.

Metallkomplexe der Formel I bestehen aus dem zugrundeliegenden organischen Molekül und einem anorganischen oder organischen Metallsalz: den Halogeniden, Nitraten, Sulfaten, Phosphaten, Acetaten, Trifluoracetaten, Trichloracetaten, Propionaten, Tartraten, Sulfonaten, Salicylaten oder Benzoaten der Elemente der dritten und vierten Hauptgruppe wie Aluminium, Zinn oder Blei sowie der ersten bis achten Nebengruppe: Chrom, Mangan, Eisen, Kobalt, Nickel, Kupfer, Zink, Silber oder Quecksilber. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d.h. sie können ein oder mehrere organische Molekülanteile als Liganden enthalten. Komplexe mit den Metallen Kupfer, Zink, Mangan und Zinn sind bevorzugt.

Aus der Literatur sind 1-Hydroxyäthyl-azol-Derivate als Pflanzenwachstumsregulatoren und Fungizide beispielsweise aus der europäischen Patentanmeldungen 40 345 und 61 835 bekannt.

Die erfindungsgemässen Verbindungen der Formel I sind bei Raumtemperatur stabile Öle, Harze oder überwiegend Feststoffe, die sich durch sehr wertvolle mikrobizide und wuchsregulierende Eigenschaften

auszeichnen. Sie lassen sich auf dem Agrarsektor oder verwandten Gebieten präventiv und kurativ zur Bekämpfung von pflanzenschädigenden Mikroorganismen und zur Regulierung des Pflanzenwuchses einsetzen, dabei sind die Triazolylmethylderivate im Umfang der Formel I bevorzugt. Die erfindungsgemässen Wirkstoffe der Formel I zeichnen sich durch eine sehr gute Verträglichkeit bei Kulturpflanzen aus.

Aufgrund ihrer ausgeprägten wuchsregulierenden und/oder mikrobiziden Wirkung sind diejenigen Verbindungen bevorzugt, in denen

$R_1$ für 1H-1,2,4-Triazol-1-yl steht, oder

$R_4$ für tert.-Butyl steht, oder

$R_6$ Wasserstoff, Methyl, Benzyl, Acetyl, Methylcarbamoyl, N,N-Dimethylcarbamoyl, N-methoxy-N-methylcarbamoyl, Methoxycarbonyl, Äthoxycarbonyl, Methylsulfonyl, 4-Tolylsulfonyl, Methylsulfamoyl oder N,N-Dimethylsulfamoyl bedeuten.

Durch Kombination einiger Untergruppen ergibt sich ein besonders bevorzugte Gruppe von Verbindungen. Diese Gruppe enthält Verbindungen, in denen

$R_1$ für 1H-1,2,4-Triazol-1-yl steht,

$R_2$ $C_1$-$C_4$-Alkyl und $R_3$ Wasserstoff oder $C_1$-$C_4$-Alkyl bedeuten,

$R_4$ für $C_1$-$C_6$-Alkyl oder für gegebenenfalls einfachs durch Halogen, Cyan, Nitro, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy oder $C_1$-$C_4$-Halogenalkylthio substituiertes Phenyl steht,

$R_5$ für $C_1$-$C_8$-Alkyl, $C_3$-$C_8$-Cycloalkyl, Phenylalkyl mit 1-4 Kohlenstoffatomen im Alkylteil oder gegebenenfalls ein- oder mehrfach durch Halogen, Cyan, Nitro, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy oder $C_1$-$C_4$-Halogenalkylthio substituiertes Phenyl steht und

$R_6$ Wasserstoff, Methyl, Benzyl, Acetyl, Methylcarbamoyl, N,N-Dimethylcarbamoyl, N-Methoxy-N-methylcarbamoyl, Methoxycarbonyl, Äthoxycarbonyl, Methylsulfonyl, 4-Tolylsulfonyl, Methylsulfamoyl oder N,N-Dimethylsulfamoyl bedeutet.

Als besonders bevorzugte Untergruppe sind diejenigen Verbindungen der Formel I anzusehen, in denen $R_1$ für 1H-1,2,4-Triazol-1-yl, $R_2$ für Methyl oder Äthyl, $R_3$ Wasserstoff, $R_4$ für iso-Propyl oder tert.-Butyl, $R_5$ für unsubstituiertes oder ein- bis zweimal durch Methyl, Nitro, Fluor, Chlor oder Brom substituiertes Phenyl, X für Sauerstoff und $R_6$ für Wasserstoff oder Methyl stehen.

Folgende Einzelverbindungen sind bevorzugt:

1-(4-Chlorphenoxy)-2-tert.butyl-2-hydroxy-3-methyl-3-(1H-1,2,4-triazol-1-yl)-propan,
1-(4-Fluorphenoxy)-2-tert.butyl-2-hydroxy-3-methyl-3-(1H-1,2,4-triazol-1-yl)-propan,
1-Phenoxy-2-tert.butyl-2-methoxy-3-methyl-3-(1H-1,2,4-triazol-1-yl)-propan,
1-Phenoxy-2-tert.butyl-2-hydroxy-3-äthyl-3-(1H-1,2,4-triazol-1-yl)-propan,
1-(4-Toluyloxy)-2-tert.butyl-2-hydroxy-3-methyl-3-(1H-1,2,4-triazol-1-yl)-propan,
1-(4-Fluorphenoxy)-2-tert.butyl-2-hydroxy-3-äthyl-3-(1H-1,2,4-triazol-1-yl)-propan,
1-(2-Fluorphenoxy)-2-tert.butyl-2-hydroxy-3-methyl-3-(1H-1,2,4-triazol-1-yl)-propan
1-(4-Nitrophenoxy)-2-tert.butyl-2-hydroxy-3-methyl-3-(1H-1,2,4-triazol-1-yl)-propan
1-(2,3-Dimethylphenoxy)-2-tert.butyl-2-hydroxy-3-methyl-3-(1H-1,2,4-triazol-1-yl)-propan,
1-(4-Chlor-2-methylphenoxy)-2-tert.butyl-2-hydroxy-3-methyl-3-(1H-1,2,4-triazol-yl)-propan,
1-(4-Bromphenoxy)-2-isopropyl-2-hydroxy-3-methyl-3-(1H-1,2,4-tria-zol-1-yl)-propan,
1-(4-Fluorphenoxy)-2-isopropyl-2-hydroxy-3-methyl-3-(1H-1,2,4-triazol-1-yl)-propan und
1-(4-Bromphenoxy)-2-tert.butyl-2-hydroxy-3-methyl-3-(1H-1,2,4-triazol-1-yl)-propan.

Die Verbindungen der Formel I werden erhalten, indem man ein Azolylmethyloxiran der Formel II

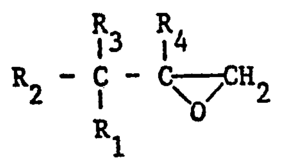

(II) ,

worin $R_1$, $R_2$, $R_3$ und $R_4$ die unter Formel I angegebene Bedeutung haben, in Gegenwart einer Base in einem inerten Lösungsmittel mit einem Alkohol oder Thioalkohol der Formel III

$$H{-}X{-}R_5 \qquad (III),$$

worin X und $R_5$ die unter Formel I gegebene Bedeutung haben, umsetzt und das Produkt der Formel Ia

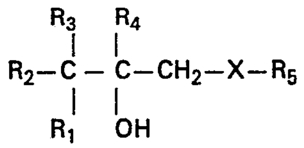

(Ia)

gewünschtenfalls durch Umsetzung mit einem Verätherungs- oder Veresterungsmittel der Formel IV

$Y-R_6$ (IV)

worin $R_6$ die unter Formel I gegebene Bedeutung hat und Y ein Halogenatom oder einen organischen oder anorganischen Säurerest bedeutet, veräthert oder verestert.

Die Reaktion (II mit III) wird vorteilhafterweise in Gegenwart von katalytischen Mengen an Basen als Kondensationsmittel durchgeführt. Als solche kommen organische und anorganische Basen in Betracht, z. B. tertiäre Amine wie Trialkylamine (Trimethylamin, Triäthylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, LiOH, $CaH_2$, KOH, NaH $Ca(OH)_2$, $KHCO_3$, $NaHCO_3$, $Ca(HCO_3)$, $K_2CO_3$, $Na_2CO_3$), sowie Alkaliacetate wie $CH_3COONa$ oder $CH_3COOK$. Darüberhinaus eignen sich auch Alkalialkoholate wie $C_2H_5ONa$, $C_3H_7$-nONa, $(CH_3)_3C$-OK usw..

Die Reaktion (II mit III) wird bevorzugt in einem relativ polaren, jedoch reaktionsinerten organischen Lösungsmittel, durchgeführt, z. B. N,N-Dimethylformamid, N,N-Dimethylacetamid, Dimethylsulfoxid, Acetonitril, Benzonitril, Äthylenglykoldimethyläther, Diäthylenglykoldimethyläther, Triäthylenglykoldimethyläther, Dioxan, Tetrahydrofuran und anderen. Derartige Reaktionen können aber auch in Kombination mit anderen reaktionsinerten Lösungsmitteln, z. B. Benzol, Toluol, Xylol, Hexan, Petroläther, Chlorbenzol, Nitrobenzol u.a. durchgeführt werden. Die Reaktionstemperaturen liegen in einem Temperaturbereich von 20° bis 250°C, vorzugsweise 80° bis 180°C.

Die fakultative Umsetzung der Verbindungen der Unterformel Ia zu denjenigen Verbindungen der Formel I, in denen $R_6$ eine andere Bedeutung als Wasserstoff hat, wird vorteilhafterweise in einem inerten organischen Lösungsmittel durchgeführt. Geeignete Lösungsmitte sind aprotische Lösungsmittel wie N,N-Dimethylformamid, N,N-Dimethylacetamid, Dimethylsulfoxid, Acetonitril, Benzonitril, N-Methylpyrrolidinon, N-Methylpiperidinon, Benzol, Toluol, Xylol, Hexan, Cyclohexan, Chlorbenzol, Nitrobenzol und andere.

Üblicherweise steht Y für den Fall, dass die -O-$R_6$-Gruppe eine Äthergruppe ist, für Halogen wie Chlor, Brom und Jod oder für Säurereste, die sich von starken Säuren ableiten wie Schwefelsäure, Phosphorsäure, Sulfonsäuren, vorzugsweise Halogenalkylsulfonsäuren oder Halogenalkancarbonsäuren wie Trifluoressigsäure. Typische Vertreter solcher Säure-Derivate sind Dimethylsulfat, Diäthylsulfat und Trifluormethansulfonsäuremethylester. Für den Fall, dass die -O-$R_6$-Gruppe eine Estergruppe ist, steht Y im allgemeinen für Halogen wie Chlor oder Brom oder für Säurereste von Säuren, die mit dem übertragenen Acylrest ein Anhydrid bilden können. Vorzugsweise handelt es sich dabei um Anhydride mit der gleichen Säure. Dem Reagenz Y-$R_6$ entspricht dann also z. B. Essigsäureanhydrid, Propionsäureanhydrid, Benzoesäureanhydrid, Benzolsulfonsäureanhydrid oder Trifluorsulfonsäureanhydrid.

Die Verätherung oder Veresterung der Verbindungen der Unterformel Ia geschieht mit Vorteil in Gegenwart von Basen wie Alkoholaten, Hydroxiden, Hydriden, Carbonaten oder Hydrogencarbonaten von Alkali- oder Erdalkalimetallen. Die Reaktionstemperaturen betragen 20-150°C, vorzugsweise 60-120°C.

Die Verbindungen der Formel I fallen als Diastereomerengemische an. Gegenstand der Erfindung sind alle diastereomeren Formen der Wirkstoffe der Formel I und Gemische davon. Umfasst sind somit sowohl die reinen Diastereomeren als auch die einzelnen optischen Isomeren der umfassten Enantiomerenpaare.

Die Alkohole oder Thioalkohole der Formel III sowie Verätherungs- und Veresterungsmittel der Formel IV sind bekannt oder werden nach an sich bekannten Methoden hergestellt.

Die Oxirane der Formel II sind neu, sie stellen besonders entwickelte Zwischenprodukte zur Herstellung der wertvollen Wirkstoffe der Formel I dar. Aufgrund ihrer strukturellen Beschaffenheit lassen sie sich in einfacher Weise in die Verbindungen der Formel I überführen.

Die Oxirane der Fornel II haben wie die Endprodukte der Formel I wuchsregulierende und mikrobizide Eigenschaften. Sie können daher auch aus Wirkstoffe in entsprechenden agrochemischen Mitteln verwendet werden. Solche Mittel bilden einen weiteren Gegenstand der vorliegenden Erfindung.

Die Oxirane der Formel II lassen sich durch Reaktion der zugrundeliegenden Ketone der Formel V

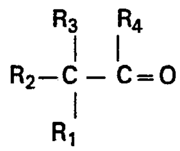 (V)

worin $R_1$, $R_2$, $R_3$ und $R_4$ die unter Formel I gegebenen Bedeutungen haben, mit Dimethylsulfoniummethylid, Dimethyloxosulfoniummethylid oder den entsprechenden Salzen wie Trimethylsulfoniumjodid oder Trimethyloxosulfoniumjodid in Gegenwart starker Basen wie Alkali- und Erdalkalialkoholate, Alkalihydroxide oder Alkali- oder Erdalkalihydride in Dimethylsulfoxid oder einen der anderen bei der Reaktion von II mit III beschriebenen Lösungsmittel herstellen. Unter geeigneten Umständen ist die Erzeugung des Sulfoniumylids bzw. die Reaktion des Sulfoniumsalzes mit der Base auch im Phasentransfer-Verfahren durchführbar. Als geeignete Phasentransfer-Katalysatoren wären verwendbar: quaternäre Ammoniumsalze wie Trialkylphenylalkylammoniumsalze oder Tetraalkylammoniumsalze, quaternäre Phosphoniumsalze wie Tetraalkylphosphoniumsalze oder Kronenäther wie 15-Krone-5 oder 18-Krone-6. Bei dieser Reaktion wird das Sulfoniumylid in situ erzeugt und reagiert direkt mit dem Keton der Formel V zum Oxiran der Formel II. Die Reaktion wird bei Temperaturen von 0° bis 120°C durchgeführt.

Analoge Reaktionen sind aus der Literatur bekannt; vgl. JACS, 87, 1353 (1965). Man kann die Reaktion prinzipiell analog zu den dort beschriebenem Umsetzungen vornehmen. Ketone der Formel V können aus den an sich bekannten α-Halogenketonen der Formel VI

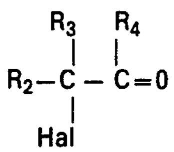 (VI),

worin $R_2$, $R_3$ und $R_4$ die unter Formel I gegebene Bedeutung haben und Hal für Chlor oder Brom steht, durch Umsetzung mit Azolen der Formel VII

$$H-R_1 \qquad (VII),$$

worin $R_1$ die unter Formel I gegebene Bedeutung hat, in Gegenwart einer Base umsetzt.

Die Ketone der Formel V können auch erhalten werden indem man ein Azolylmethylketon der Formel VIII

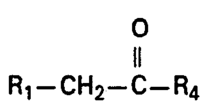 (VIII)

in Gegenwart einer Base mit Verbindungen der Formel IX und/oder X

$$Z-R_2 \quad (IX), \qquad Z-R_3 \quad (X),$$

worin $R_2$ und $R_3$ die unter Formel I gegebenen Bedeutungen haben und Z für ein Halogenatom oder einen organischen oder anorganischen Säurerest steht, umsetzt.

Die Herstellung der Ketone der Formel V erfolgt in den üblichen inerten Lösungsmitteln und gegebenenfalls bei erhöhter Temperatur.

Die Verbindungen der Formeln VI, VII, VIII, IX und X sind bekannt und zum Teil im Handel erhältlich oder können nach bekannten Methoden hergestellt werden.

Bei der Herstellung aller hierin genannten Ausgangs-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petroläther; halogenierte Kohlenwasserstoffe wie Chlorbenzol, Methylenchlorid, Äthylenchlorid, Chloroform, Tetrachlorkohlenstoff, Tetrachloräthylen; Äther und ätherartige Verbindungen wie Dialkyläther (Diäthyläther, Diisopropyläther, tert.-Butylmethyläther usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diäthylketon, Methyläthylketon und Gemische solcher Lösungsmittel untereinander. In manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon, oder in gewissen Fällen auch Kohlendioxid.

Das beschriebene Herstellungsverfahren ist, einschliesslich aller Teischritte, ein wichtiger Bestandteil der vorliegenden Erfindung.

Es wurde nun überraschend gefunden, dass die neuen Wirkstoffe der Formel I bzw. Mittel, die diese Wirkstoffe enthalten, sich vor allem dadurch auszeichnen, dass sie gezielt in den Metabolismus der Pflanzen eingreifen. Dieser gezielte Eingriff in die physiologischen Vorgänge der Pflanzenentwicklung macht die

Wirkstoffe der Formel I für verschiedene Zwecke verwendbar, insbesondere für solche, die mit der Ertragssteigerung bei Nutzpflanzen, der Ernteerleichterung und der Arbeitseinsparung bei Massnahmen an Pflanzenkulturen im Zusammenhang stehen.

Für die Wirkungsweise von Pflanzenwachstumsregulatoren gilt nach der bisherigen Erfahrung, dass ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn dadurch kann z. B. die Häufigkeit der Grasschnitte in Ziergärten, Park- und Sportanlagen oder an Strassenrändern reduziert werden. Von Bedeutung ist auch die Hemmung des Wuchses von krautigen und holzigen Pflanzen an Strassenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide denn durch eine Halmverkürzung wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, so dass z. B. mehr oder grössere Früchte zur Ausbildung kommen. Ertragssteigerungen können in manchen Fällen auch durch einen Eingriff in den pflanzlichen Stoffwechsel, wie z. B. durch Erhöhung der Photosyntheseleistung, erreicht werden, ohne dass sich Änderungen des vegetativen Wachstums bemerkbar machen. Wachstumsregulatoren können ferner eine Veränderung der Zusammensetzung der Pflanzen bewirken, so dass eine bessere Qualität der Ernteprodukte herbeigeführt wird. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluss von Wachstumsregulatoren kann es zur Ausbildung parthenokarper Früchte kommen. Ferner kann das Geschlecht der Blüten beeinflusst werden.

Mit Wachstumsregulatoren lässt sich auch die Produktion oder der Abfluss von sekundären Pflanzenstoffen positiv beeinflussem. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z. B. Interesse bei der Stecklingsvermehrung von Pflanzen. Es ist jedoch auch möglich, das Wachstum der Seitentriebe zu hemmen, z. B. um bei Tabakpflanzen nach der Dekapitierung die Ausbildung von Seitentrieben zu verhindern und damit das Blattwachstum zu fördern.

Durch Einsatz von Wachstumsregulatoren lässt sich der vorzeitige Fruchtfall verhindern. Es ist jedoch auch möglich, den Fruchtfall, - zum Beispiel bei Obst -, im Sinne einer chemischen Ausdünnung bis zu einem bestimmten Ausmass zu fördern. Wachstumsregulatoren können auch dazu dienen um bei Kulturpflanzen zum Zeitpunkt der Ernte die zum Ablösen der Früchte erforderliche Kraft zu vermindern, so dass eine mechanische Beerntung der Pflanzen ermöglicht, beziehungsweise eine manuelle Beerntung erleichtert wird.

Mit Wachstumsregulatoren lässt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darübeihinaus kann mit Hilfe von Wachstumsregulatoren auch eine zeitliche Konzentrierung der Reife erzielt werden. Damit werden Voraussetzungen dafür geschaffen, dass z. B. bei Tabak, Tomaten oder Kaffee, eine vollständige mechanische oder manuelle Beerntung in nur einem Arbeitsgang vorgenommen werden kann.

Durch die Anwendung von Wachstumsregulatoren kann auch die Samen- oder Knospenruhe der Pflanzen, also die endogene Jahresrhythmik, beeinflusst werden, so dass die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, dass der Austrieb von Knospen oder die Keimung von Samen verzögert wird, z. B. um in frostgefährdeten Gebieten eine Schädigung durch Spätfröste zu vermeiden. Andererseits gelingt es, das Wurzelwachstum zu und/oder die Ausbildung von Sprösslingen zu stimulieren, so dass das Wachstum auf eine kürzere Zeitdauer beschränkt werden kann.

Wachstumsregulatoren können auch eine Halophilie bei den Kulturpflanzen erzeugen. Damit werden die Voraussetzungen dafür geschaffen, dass eine Kultivierung von Pflanzen auf salzhaltigen Böden durchgeführt

werden kann.

Mit Wachstumsregulatoren kann auch eine Frost- und Trockenresistenz bei Pflanzen induziert werden.

Unter dem Einfluss von Wachstumsregulatoren kann das Altern (die Seneszenz) von Pflanzen oder Pflanzenteilen gehemmt respektive verzögert werden. Eine solche Wirkung kann von hohem wirtschaftlichem Interesse sein, dadurch, dass bei behandelten Pflanzenteilen oder ganzen Pflanzen wie Obst, Beeren, Gemüse, Salat oder Zierpflanzen deren Lagerfähigkeit nach der Ernte verbessert oder verlängert werden kann. Ebenso kann durch Behandlung von Kulturpflanzen über eine Verlängerung der Phase photosynthetischer Aktivität eine beachtliche Ertragssteigerung erzielt werden.

Ein weiteres wichtiges Anwendungsgebiet für Wuchshemmer ist deren Einsatz zur Hemmung eines übermässigen Wachstums bei tropischen Bodenbeckungspflanzen, den sogenannten Cover crops. In tropischen und subtropischen Monokulturen, wie z. B. in Palmplantagen, Baumwoll-, Maisfeldern usw. werden neben den eigentlichen Kulturpflanzen oftmals Bodenbedeckungspflanzen, insbesondere Leguminosenarten angepflanzt, die zur Erhaltung oder Steigerung der Bodenqualität (Verhinderung der Austrocknung, Versorgung mit Stickstoff) und zur Verhinderung von Erosion (Abträgung durch Wind und Wasser) dienen. Durch Applikation der erfindungsgemässen Wirkstoffe kann nunmehr das Wachstum dieser Cover crops kontrolliert und somit die Wuchshöhe dieser Bodenbedeckungspflanzen auf einem niedrigen Niveau gehalten werden, so dass ein gesundes Gedeihen der Kulturpflanzen und die Aufrechterhaltung einer günstigen Bodenbeschaffenheit gewährleistet ist.

Weiter hat es sich überraschenderweise gezeigt, dass die Aktivsubstanzen der Formel I bzw. entsprechende Mittel ausser vorteilhaften wuchsregulierenden Eigenschaften zusätzlich ein für praktische Bedürfnisse sehr günstiges Mikrobizidspektrum aufweisen. Deshalb liegt ein weiteres Einsatzgebiet von Verbindungen der Formel I in der Bekämpfung von schädlichen Mikroorganismen, vor allem von phytopathogenen Pilzen. So besitzen die Verbindungen der Formel I eine für praktische Bedürfnisse sehr günstige kurative, präventive und systemische Wirkung zum Schutz von Pflanzen, insbesondere Kulturpflanzen, ohne diese nachteilig zu beeinflussen.

Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z. B. Venturia, Podosphaera, Erysiphe, Monilinia, Uncinula); Basidiomyceten (z. B. die Gattungen Hemileia, Rhizoctonia, Puccinia); Fungi imperfecti (z. B. Bortrytis, Helminthosporium, Fusarium, Septoria, Cercospora und Alternaria). Überdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten: Getreide: (Weizen, Gerste, Roggen, Hafer, Reis, Sorhum und Verwandte); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer): oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedeckungen (cover crops), die einer Erosion oder Austrocknung des Bodens entgegenwirken oder Bodenbedeckungen wie sie in Baum- und Staudenkulturen (Obstplantagen, Hopfenkulturen, Maisfeldern, Weingärten usw.) erwünscht sind.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusatzen.

Geeignete Träger und Zusätze können fest oder flüssig sein und entsprechen den in der

Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen richten sich dabei nach dem Befallsdruck für den entsprechenden Erreger (Pilzsorte) oder der Art der Wachstumsbeeinflussung. Die Wirkstoffe der Formel I können aber auch über den Erdboden durch das Wurzelwerk in die Pflanzen gelangen (systemische Wirkung), indem man den Standort der Pflanzen mit einer flüssigen Zubereitung tränkt oder die Substanzen in fester Form in den Boden einbringt, z. B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z. B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha; bevorzugt 100 g bis 2 kg AS/ha, insbesondere bei 200 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidom, Dimethylsulfoxid oder Dimethylformamid, sowie gegebenemfalls epoxidiertes Pflanzenöl wie epoxydiertes Kokosnussöl oder Soja, Öl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden.

Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Öl- oder Stearinsäure, oder von natürlichen Fettsäuregemischen, die z. B. aus Kokonuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes und Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propypenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)äthylammonium-bromid.

Die in der Formulierungstechnik gebräuchlichen Tenside sind u.a. in folgenden Publikationen beschrieben:
"Mc Cutcheon"s Detergents and Emulsifiers Annual" MC Publishing Corp., Ridgewood, New Jersey, 1979.
Sisley and Wood, "Encyclopedia of Surface Active Agents", Chemical Publishing Co., Inc. New York, 1964.

Die pestiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 %, eines Tensides.

Insbesondere setzen sich bevorzugte Formulierungen folgendermassen zusammen: (% = Gewichtsprozent)

| | |
|---|---|
| Lösungen | |
| Aktiver Wirkstoff: | 5 bis 95 %, vorzugsweise 10 bis 80 % |
| Lösungsmittel: | 95 bis 5 %, vorzugsweise 90 bis 0 % |
| oberflächenaktives Mittel: | 1 bis 30 %, vorzugsweise 2 bis 20 %. |
| Emulgierbare Konzentrate | |
| Aktiver Wirkstoff | 10 bis 50 %, bevorzugt 10 bis 40 %. |
| oberflächenaktives Mittel: | 5 bis 30 %, vorzugsweise 10 bis 20 % |
| flüssiges Trägermittel: | 20 bis 95 %, vorzugsweise 40 bis 20 %. |
| Stäube | |
| Aktiver Wirkstoff: | 0,5 bis 10 %, vorzugsweise 2 bis 8 % |
| festes Trägermaterial | 99,5 bis 90 %, vorzugsweise 98 bis 92 %. |
| Suspensions-Konzentrate | |
| Aktiver Wirkstoff | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |
| Benetzbare Pulver | |
| Aktiver Wirkstoff: | 5 bis 90 %, vorzugsweise 10 bis 80 % und insbesondere 20 bis 60 % |
| oberflächenaktives Mittel: | 0,5 bis 20 %, vorzugsweise 1 bis 15 % |
| festes Trägermaterial: | 5 bis 90 %, vorzugsweise 30 bis 70 %. |
| Granulate: | |
| Aktiver Wirkstoff: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe zu beschränken. Temperaturen sind in Celsiusgraden angegeben.

**Herstellungsbeispiele**

**Biepsiel 1:**

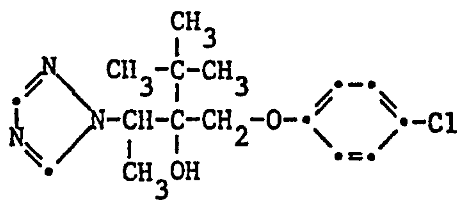

1-(4-Chlorphenoxy)-2-tert.butyl-2-hydroxy-3-methyl-3-(1H-1,2,4-triazol-1-yl)-propan (Verbindung Nr. 1).

a) 2-tert.Butyl-2-[1-(1H-1,2,4-triazol-1-yl)-äthyl]-oxiran:

Ein Gemisch aus 23 g (0,127 Mol) 1-(1H-1,2,4-Triazol-1-yl)-äthyl-tert.-butyl-keton, 33 g (0,15 Mol) Trimethyloxosulfoniumjodid, 1 g (0,0031 Mol) Tetrabutylammoniumbromid, 30 ml wässrige 50 %ige Kaliumhydroxidlösung und 80 ml Toluol wird unter Rühren für 24 Stunden auf 90°C erwärmt. Nach dem Abkühlen wird die organische Phase abgetrennt, mit Wasser gewaschen, getrocknet und eingedampft. Man erhält als Rückstand 16,0 g (96 % d.Th.) 2-tert.Butyl-2-[1-(1H-1,2,4-triazol-1-yl)-äthyl]-oxiran, Sdp. 68-70°C, 0,01 mbar. (Verbindung Nr. 401)

b) Ein Gemisch aus 16 g (0,082 Mol) 2-tert.Butyl-2-[1-(1H-1,2,4-triazol-1-yl)-äthyl]-oxiran, 12,6 g (0,106 Mol) 4-Chlorphenol, 1 g (0,024 Mol) Lithiumhydroxidhydrat und 50 ml Diäthylenglykoldimethyläther wird für 12 Stunden auf 150°C erhitzt. Nach dem Abkühlen wird das Reaktiomsgemisch in Eiswasser gegossen und mit Äthylacetat extrahiert. Durch Trocknen und Eindampfen der organischen Phase erhält man 12 g (51,3 % d.Th.) 1-(4-Chlorphenoxy)-2-tert.butyl-2-hydroxy-3-methyl-3-(1H-1,2,4-triazol-1-yl)-propan als 1:1-Diastereomerengemisch, Smp. 80°-104°C (Verbindung Nr. 1).

Analyse: $C_{16}H_{22}ClN_3O$

| | | | | |
|---|---|---|---|---|
| ber: | C 59,35 % | H 6,85 % | N 12,98 % | Cl 10,85 % |
| gef: | C 59,6 % | H 7,0 % | N 12,8 % | Cl 10,5 % |

Die Trennung der Diastereomeren gelingt durch Chromatogrophie an Kieselgel mit einem 4:1-Gemisch aus Äthylacetat und Toluol. Man erhält 2 Diastereomere mit den Schmelzpunkten 109°-110°C (Verbindung Nr. 2), resp. 128°-130°C (Verbindung Nr. 3).

**Beispiel 2**

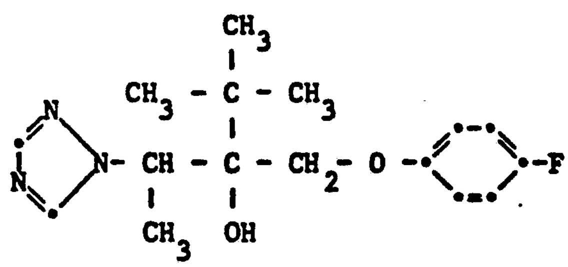

1-(4-Fluorphenoxy)-2-tert.butyl-2-hydroxy-3-methyl-3-(1H-1,2,4-triazol-1-yl)-propan

Ein Gemisch von 10,0 g 2-tert.Dutyl-2-[1-(1H-1,2,4-triazol-1-yl)-äthyl]-oxiran, 6,4 g 4-Fluorphenol und 0,1 g Lithiumhydroxidhydrat wird in 10 ml Diäthylenglykoldimethyläther für 18 Stunden auf 150°C erhitzt. Nach dem Abkühlen wird das Reaktionsgemisch mit Eiswasser aufgenommen und mit Äther extrahiert. Die organische Phase wird mit Wasser und gesättigter Kochsalzlösung gewaschen, getrocknet und eingedampft. Durch Chromatographie an Kieselgel mit Äthylacetat/Toluol-Gemisch (3:1) erhält man 11,0 g 1-(4-Fluorphenoxy)-2-tert.butyl-2-hydroxy-3-methyl-3-(1H-1,2,4-triazol-1-yl)-propan,

Analyse: $C_{16}H_{22}FN_3O_2$

| | | | | |
|---|---|---|---|---|
| berechnet | C 62,52 % | H 7,22 % | N 13,67 % | F 6,18 % |
| gefunden | C 62,6 % | H 7,2 % | N 13,7 % | F 6,3 % |

**Beispiel 3**:

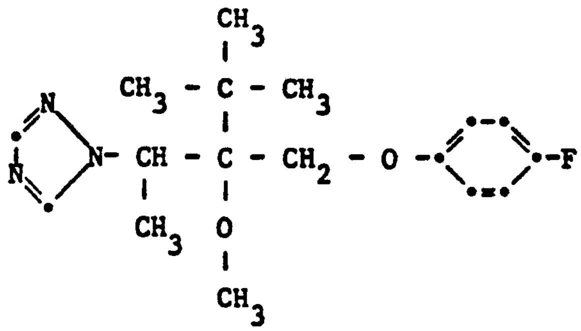

1-(4-Fluorphenoxy)-2-tert.butyl-2-methoxy-3-methyl-3-(1H-1,2,4-triazol-1-yl)-propan.

1,7 g 52 %iges Natriumhydrid werden in Hexan dispergiert. Nach dem Absetzen wird das überschüssige Hexan abgetrennt und die Natriumhydriddispersion in 100 ml Tetrahydrofuran aufgenommen. Zu dieser Mischung werden 10,0 g des nach Beispiel 3 erhaltenen 1-(4-Fluorphenoxy)-2-tert.butyl-2-methoxy-3-methyl-3-(1H-1,2,4-triazol-1-yl)-propans zugesetzt. Anschliessend wird das Reaktionsgemisch auf 60°C erwärmt und solange bei dieser Temperatur erhalten bis kein Wasserstoffgas mehr entwickelt wird. Anschliessend setzt man 5,7 g Methyljodid zu und rührt das Gemisch für 18 Stunden bei 20 - 25°C. Nach Aufnehmen mit Eiswasser und Extrahieren mit Äthylacetat wird die organische Phase mit Wasser und gesättigter Kochsalzlösung gewaschen, getrocknet und eingedampft. Nach Chromatographie des Rückstandes an Kieselgel mit Hexan/Äthylacetat-Gemisch erhält man als gelbes hochviskoses Öl 7,2 g 1-(4-Fluorphenoxy)-2-tert.butyl-2-methoxy-3-methyl-3-(1H-1,2,4-triazol-1-yl)-propan

In analoger Weise werden die in den folgenden Tabellen aufgeführten Verbindungen hergestellt.

**Tabelle 1**:

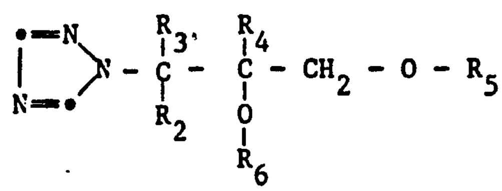

| Nr. | $R_2$ | $R_3$ | $R_4$ | $R_5$ | $R_6$ | phys. Daten |
|---|---|---|---|---|---|---|
| 1 | $CH_3$ | H | t-$C_4H_9$ | 4-Cl-$C_6H_4$- | H | Smp. 90-104°C |
| 2 | $CH_3$ | H | t-$C_4H_9$ | 4-Cl-$C_6H_4$- | H | Diastereomes I Smp. 109-110°C |
| 3 | $CH_3$ | H | t-$C_4H_9$ | 4-Cl-$C_6H_4$- | H | Diastereomes II Smp. 128-130°C |
| 4 | $CH_3$ | H | t-$C_4H_9$ | 4-Cl-$C_6H_4$- | $CH_3$ | hochviskoses Öl |
| 5 | $CH_3$ | H | t-$C_4H_9$ | 4-Cl-$C_6H_4$- | -CO-$CH_3$ | |
| 6 | $CH_3$ | H | t-$C_4H_9$ | 4-Cl-$C_6H_4$- | -$CH_2$-$C_6H_5$ | |
| 7 | $CH_3$ | H | t-$C_4H_9$ | 4-Cl-$C_6H_4$- | -CO-N$(CH_3)_2$ | |
| 8 | $CH_3$ | H | t-$C_4H_9$ | 4-Cl-$C_6H_4$- | -CO-N$(CH_3)OCH_3$ | |
| 9 | $CH_3$ | H | t-$C_4H_9$ | 4-Cl-$C_6H_4$- | -$SO_2$-$CH_3$ | |
| 10 | $CH_3$ | H | t-$C_4H_9$ | 4-Cl-$C_6H_4$- | -$SO_2$-N$(CH_3)_2$ | |
| 11 | $CH_3$ | H | t-$C_4H_9$ | 4-Cl-$C_6H_4$- | -CO-$C_2H_5$ | |
| 12 | $CH_3$ | H | t-$C_4H_9$ | 4-Cl-$C_6H_4$- | -CO-$C_3H_7$-n | |

| | | | | | | |
|---|---|---|---|---|---|---|
| 13 | $CH_3$ | H | $t-C_4H_9$ | $4-F-C_6H_4-$ | H | Smp. 85-100°C |
| 14 | $C_2H_5$ | H | $t-C_4H_9$ | $4-F-C_6H_4-$ | H | Öl |
| 15 | $C_2H_5$ | H | $t-C_4H_9$ | $4-Cl-C_6H_4-$ | H | Smp. 97-99° |
| 16 | $CH_3$ | H | $t-C_4H_9$ | $4-F-C_6H_4-$ | $CH_3$ | hochviskoses Öl |
| 17 | $CH_3$ | H | $t-C_4H_9$ | $4-F-C_6H_4-$ | $-CO-CH_3$ | |
| 18 | $CH_3$ | H | $t-C_4H_9$ | $4-F-C_6H_4-$ | $-CH_2-C_6H_5$ | |
| 19 | $CH_3$ | H | $t-C_4H_9$ | $4-F-C_6H_4-$ | $-CO-NHCH_3$ | |
| 20 | $CH_3$ | H | $t-C_4H_9$ | $4-Cl-C_6H_4-$ | $-CO-NHCH_3$ | |
| 21 | $CH_3$ | H | $t-C_4H_9$ | $4-F-C_6H_4-$ | $-CO-N(CH_3)_2$ | |
| 22 | $CH_3$ | H | $t-C_4H_9$ | $4-F-C_6H_4-$ | $-CO-N(CH_3)OCH_3$ | |
| 23 | $CH_3$ | H | $t-C_4H_9$ | $4-F-C_6H_4-$ | $-SO_2-CH_3$ | |
| 24 | $CH_3$ | H | $t-C_4H_9$ | $4-F-C_6H_4-$ | $-SO_2-N(CH_3)_2$ | |
| 25 | $CH_3$ | H | $t-C_4H_9$ | $4-F-C_6H_4-$ | $-CO-C_2H_5$ | |
| 26 | $CH_3$ | H | $t-C_4H_9$ | $4-F-C_6H_4-$ | $-CO-C_3H_7-n$ | |
| 27 | $CH_3$ | H | $t-C_4H_9$ | $4-Br-C_6H_4-$ | H | Smp. 90-98°C |
| 28 | $C_2H_5$ | H | $t-C_4H_9$ | $4-Br-C_6H_4-$ | H | Öl |
| 29 | $C_2H_5$ | $CH_3$ | $t-C_4H_9$ | $4-Br-C_6H_4-$ | H | |
| 30 | $C_2H_5$ | $CH_3$ | $t-C_4H_9$ | $4-F-C_6H_4-$ | H | |
| 31 | $C_2H_5$ | $CH_3$ | $t-C_4H_9$ | $4-Cl-C_6H_4-$ | H | |
| 32 | $CH_3$ | H | $t-C_4H_9$ | $4-Br-C_6H_4-$ | $CH_3$ | Öl |
| 33 | $CH_3$ | H | $t-C_4H_9$ | $4-Br-C_6H_4-$ | $-CO-CH_3$ | |
| 34 | $CH_3$ | H | $t-C_4H_9$ | $4-Br-C_6H_4-$ | $-CH_2-C_6H_5$ | |
| 35 | $CH_3$ | H | $t-C_4H_9$ | $4-Br-C_6H_4-$ | $-CO-NH-CH_3$ | |
| 36 | $CH_3$ | H | $t-C_4H_9$ | $4-Br-C_6H_4$ | $-CO-N(CH_3)_2$ | |
| 37 | $CH_3$ | H | $t-C_4H_9$ | $4-Br-C_6H_4-$ | $-CO-N(CH_3)OCH_3$ | |
| 38 | $CH_3$ | H | $t-C_4H_9$ | $4-Br-C_6H_4-$ | $-SO_2-CH_3$ | |
| 39 | $CH_3$ | H | $t-C_4H_9$ | $4-Br-C_6H_4-$ | $-SO_2-N(CH_3)_2$ | |
| 40 | $CH_3$ | H | $t-C_4H_9$ | $4-Br-C_6H_4-$ | $-CO-C_2H_5$ | |
| 41 | $CH_3$ | H | $t-C_4H_9$ | $4-Br-C_6H_4-$ | $-CO-C_3H_7-n$ | |
| 42 | $CH_3$ | H | $t-C_4H_9$ | $C_6H_5-$ | H | halbkristallin |
| 43 | $CH_3$ | H | $t-C_4H_9$ | $C_6H_5-$ | $CH_3$ | |
| 44 | $CH_3$ | H | $t-C_4H_9$ | $C_6H_5-$ | $-CO-CH_3$ | |
| 45 | $CH_3$ | H | $t-C_4H_9$ | $4-CH_3-C_6H_4-$ | H | Diastereomeres II Smp.101-102°C |
| 46 | $CH_3$ | H | $t-C_4H_9$ | $2-Cl-4-Cl-C_6H_3-$ | H | Smp. 136-139°C |
| 47 | $CH_3$ | H | $t-C_4H_9$ | $2-Cl-3-Cl-C_6H_3-$ | H | Smp. 147-148°C |
| 48 | $CH_3$ | H | $t-C_4H_9$ | $4-CF_3-C_6H_4-$ | H | |
| 49 | $CH_3$ | H | $t-C_4H_9$ | $3-Cl-5-Cl-C_6H_3-$ | H | |
| 50 | $CH_3$ | H | $t-C_4H_9$ | $2-CH_3-3-CH_3-C_6H_3-$ | H | Smp. 171-172°C |
| 51 | $CH_3$ | H | $t-C_4H_9$ | $3-Cl-C_6H_4-$ | H | |
| 52 | $CH_3$ | H | $t-C_4H_9$ | $2-Cl-C_6H_4-$ | H | Smp. 143-145°C |
| 53 | $CH_3$ | H | $t-C_4H_9$ | $2-Cl-6-Cl-C_6H_3-$ | H | Smp. 101-104°C |
| 54 | $CH_3$ | H | $t-C_4H_9$ | $4-OCH_3-C_6H_4-$ | H | Smp. 88-92°C |
| 55 | $CH_3$ | H | $t-C_4H_9$ | $4-SCHF_2-C_6H_4-$ | H | |
| 56 | $CH_3$ | H | $t-C_4H_9$ | $4-OCHF_2-C_6H_4-$ | H | |
| 57 | $CH_3$ | H | $t-C_4H_9$ | $4-OCF_3-C_6H_4-$ | H | Diastereomeres I Smp. 98-99°C |
| 58 | $CH_3$ | H | $t-C_4H_9$ | $4-NO_2-C_6H_4-$ | H | Smp. 107-108°C |
| 59 | $C_2H_5$ | H | $t-C_4H_9$ | $4-Cl-C_6H_4-$ | $CH_3$ | |
| 60 | $C_2H_5$ | H | $t-C_4H_9$ | $4-Cl-C_6H_4-$ | $-CO-CH_3$ | |
| 61 | $C_2H_5$ | H | $t-C_4H_9$ | $4-Cl-C_6H_4-$ | $-CH_2-C_6H_5$ | |
| 62 | $C_2H_5$ | H | $t-C_4H_9$ | $4-F-C_6H_4-$ | $CH_3$ | |
| 63 | $C_2H_5$ | H | $t-C_4H_9$ | $4-F-C_6H_4-$ | $-CO-CH_3$ | |
| 64 | $C_2H_5$ | H | $t-C_4H_9$ | $4-F-C_6H_4-$ | $-CO-N(CH_3)_2$ | |
| 65 | $C_2H_5$ | H | $t-C_4H_9$ | $4-Br-C_6H_4-$ | $CH_3$ | |
| 66 | $C_2H_5$ | H | $t-C_4H_9$ | $4-Br-C_6H_4-$ | $-CO-CH_3$ | |
| 67 | $C_2H_5$ | H | $t-C_4H_9$ | $4-Br-C_6H_4-$ | $-CH_2-C_6H_5$ | |
| 68 | $n-C_3H_7$ | H | $t-C_4H_9$ | $4-Br-C_6H_4-$ | H | Öl |
| 69 | $n-C_3H_7$ | H | $t-C_4H_9$ | $4-Br-C_6H_4-$ | $-CO-CH_3$ | |
| 70 | $n-C_3H_7$ | H | $t-C_4H_9$ | $4-Cl-C_6H_4-$ | H | Öl |
| 71 | $n-C_3H_7$ | H | $t-C_4H_9$ | $4-Cl-C_6H_4-$ | $CH_3$ | |
| 72 | $n-C_3H_7$ | H | $t-C_4H_9$ | $4-Cl-C_6H_4-$ | $-CO-CH_3$ | |
| 73 | $n-C_3H_7$ | H | $t-C_4H_9$ | $4-F-C_6H_4-$ | H | Öl |
| 74 | $n-C_3H_7$ | H | $t-C_4H_9$ | $4-F-C_6H_4-$ | $CH_3$ | |
| 75 | $n-C_3H_7$ | H | $t-C_4H_9$ | $4-F-C_6H_4-$ | $-CH_2-C_6H_5$ | |

| | | | | | | |
|---|---|---|---|---|---|---|
| 76 | n-$C_3H_7$ | H | t-$C_4H_9$ | 4-F-$C_6H_4$- | -CO-$CH_3$ | |
| 77 | n-$C_3H_7$ | H | t-$C_4H_9$ | 4-$CH_3$-$C_6H_4$- | H | Öl |
| 78 | n-$C_3H_7$ | H | t-$C_4H_9$ | 4-$OCHF_2$-$C_6H_4$- | H | |
| 79 | n-$C_3H_7$ | H | t-$C_4H_9$ | $C_6H_5$ | H | Öl |
| 80 | n-$C_3H_7$ | H | t-$C_4H_9$ | $C_6H_5$ | $CH_3$ | |
| 81 | n-$C_4H_9$ | H | t-$C_4H_9$ | 4-Cl-$C_6H_4$- | H | |
| 82 | n-$C_4H_9$ | H | t-$C_4H_9$ | 4-Br-$C_6H_4$- | H | |
| 83 | n-$C_4H_9$ | H | t-$C_4H_9$ | 4-F-$C_6H_4$- | H | |
| 84 | n-$C_4H_9$ | H | t-$C_4H_9$ | 4-$CH_3C_6H_4$- | H | |
| 85 | n-$C_4H_9$ | H | t-$C_4H_9$ | $C_6H_5$ | H | |
| 86 | $CH_3$ | H | -$C(CH_3)_2$-$C_2H_5$ | 4-Cl-$C_6H_4$- | H | |
| 87 | $CH_3$ | H | -$C(CH_3)_2$-$C_2H_5$ | 4-Cl-$C_6H_4$- | $CH_3$ | |
| 88 | $CH_3$ | H | -$C(CH_3)_2$-$C_2H_5$ | 4-Cl-$C_6H_4$- | -CO-$CH_3$ | |
| 89 | $CH_3$ | H | -$C(CH_3)_2$-$C_2H_5$ | 4-Cl-$C_6H_4$- | -$CH_2$-$C_6H_5$ | |
| 90 | $CH_3$ | H | -$C(CH_3)_2$-$C_2H_5$ | 4-F-$C_6H_4$- | H | |
| 91 | $CH_3$ | H | -$C(CH_3)_2$-$C_2H_5$ | 4-Br-$C_6H_4$- | H | |
| 92 | $CH_3$ | H | -$C(CH_3)_2$-$C_2H_5$ | 4-$CH_3$-$C_6H_4$- | H | |
| 93 | $CH_3$ | H | i-$C_3H_7$ | 4-Cl-$C_6H_4$- | H | $n_D^{30}$ 1,5323 |
| 94 | $CH_3$ | H | i-$C_3H_7$ | 4-Cl-$C_6H_4$- | $CH_3$ | |
| 95 | $CH_3$ | H | i-$C_3H_7$ | 4-Cl-$C_6H_4$- | -CO-$CH_3$ | |
| 96 | $CH_3$ | H | t-$C_4H_9$ | n-$C_4H_9$ | H | |
| 97 | $CH_3$ | H | t-$C_4H_9$ | n-$C_4H_9$ | $CH_3$ | |
| 98 | $CH_3$ | H | t-$C_4H_9$ | n-$C_4H_9$ | -CO-$CH_3$ | |
| 99 | $CH_3$ | H | t-$C_4H_9$ | cycl-$C_6H_{11}$ | H | |
| 100 | $CH_3$ | H | t-$C_4H_9$ | cycl-$C_6H_{11}$ | $CH_3$ | |
| 101 | $CH_3$ | H | t-$C_4H_9$ | cycl-$C_6H_{11}$ | -CO-$CH_3$ | |
| 102 | $CH_3$ | H | t-$C_4H_9$ | cycl-$C_6H_{11}$ | -$CH_2$-$C_6H_5$ | |
| 103 | $CH_3$ | H | t-$C_4H_9$ | $CH_3$ | H | Smp. 149-155° C |
| 104 | $C_2H_5$ | H | t-$C_4H_9$ | $CH_3$ | H | Diastereomeres I Smp. 116 - 118° C |
| 105 | $C_2H_5$ | H | t-$C_4H_9$ | 4-Cl-$C_6H_4$- | H | Diastereomeres I Smp. 97 - 99° C |
| 106 | $CH_3$ | H | t-$C_4H_9$ | 4-Br-$C_6H_4$- | H | Diastereomeres I Smp. 128 - 130° C |
| 107 | $CH_3$ | H | t-$C_4H_9$ | 4-Br-$C_6H_4$- | H | Diastereomeres II Smp. 90 - 98° C |
| 108 | $C_2H_5$ | H | t-$C_4H_9$ | $C_6H_5$ | H | Öl |
| 109 | $C_2H_5$ | H | t-$C_4H_9$ | 4-$CH_3$-$C_6H_4$- | H | Öl |
| 110 | $CH_3$ | H | t-$C_4H_9$ | 2-F-$C_6H_4$- | H | Smp. 115-116° C |
| 111 | $CH_3$ | H | t-$C_4H_9$ | 2-$CH_3$-4-Cl-$C_6H_5$- | H | Smp. 131-132° C |
| 112 | $CH_3$ | H | t-$C_4H_9$ | 2-$CH_3$-$C_6H_4$- | H | Smp.133-134° C |
| 113 | $CH_3$ | H | t-$C_4H_9$ | 2-Br-$C_6H_4$- | H | Smp. 148-149° C |
| 114 | $CH_3$ | H | i-$C_3H_7$ | 4-Br-$C_6H_4$- | H | halbkristallin |
| 115 | $CH_3$ | H | i-$C_3H_7$ | 4-F-$C_6H_4$- | H | $n_D^{30}$: 1,5091 |
| 116 | $CH_3$ | H | t-$C_4H_9$ | 3-$CH_3$-$C_6H_4$- | H | Smp. 88-90° C |
| 117 | $CH_3$ | H | t-$C_4H_9$ | 3-Br-$C_6H_4$- | H | Smp.102-105° C |
| 118 | $CH_3$ | H | t-$C_4H_9$ | 4-$C_4H_9$-t-$C_6H_4$- | H | Smp. 92-95° C |
| 119 | $CH_3$ | H | t-$C_4H_9$ | 4-$OCF_3$-$C_6H_4$- | H | Diastereomeres II Smp.74-76° C |
| 120 | $CH_3$ | H | n-$C_6H_{13}$ | 4-Cl-$C_6H_4$- | H | $n_D^{20}$: 1,5248 |
| 121 | $C_2H_5$ | H | t-$C_4H_9$ | 4-$OCF_3$-$C_6H_4$- | H | viskoses Öl |
| 122 | $CH_3$ | H | i-$C_3H_7$ | 4-$CH_3$-$C_6H_4$- | H | Smp.68-72° C |
| 123 | $CH_3$ | H | i-$C_4H_9$ | 4-F-$C_6H_4$- | H | $n_D^{35}$: 1,5125 |
| 124 | $CH_3$ | H | i-$C_4H_9$ | 4-Cl-$C_6H_4$- | H | $n_D^{35}$: 1,5310 |
| 125 | $CH_3$ | $CH_3$ | $CH_3$ | 4-Cl-$C_6H_4$- | H | Smp. 84-86° C |
| 126 | $CH_3$ | H | -$CH(CH_3)$-$C_4H_9$-n | $C_6H_5$ | H | $n_D^{25}$: 1,5150 |
| 127 | $CH_3$ | H | -$CH(CH_3)$-$C_4H_9$-n | 4-F-$C_6H_4$- | H | $n_D^{25}$: 1,5080 |
| 128 | $CH_3$ | H | -$CH(CH_3)$-$C_4H_9$-n | 4-Cl-$C_6H_4$- | H | $n_D^{25}$: 1,5160 |
| 129 | $CH_3$ | H | -$CH(CH_3)$-$C_4H_9$-n | 4-Br-$C_6H_4$- | H | $n_D^{25}$: 1,5190 |
| 130 | $CH_3$ | H | -$CH(C_2H_5)$-$C_3H_7$-n | 4-Cl-$C_6H_4$- | H | |
| 131 | $CH_3$ | H | -$CH(C_2H_5)$-$C_3H_7$-n | 4-F-$C_6H_4$- | H | |
| 132 | $CH_3$ | H | i-$C_5H_{11}$ | 4-F-$C_6H_4$- | H | |
| 133 | $CH_3$ | H | i-$C_5H_{11}$ | 4-Cl-$C_6H_4$- | H | |
| 134 | $CH_3$ | H | i-$C_5H_{11}$ | 4-Br-$C_6H_4$- | H | |

**Tabelle 2:**

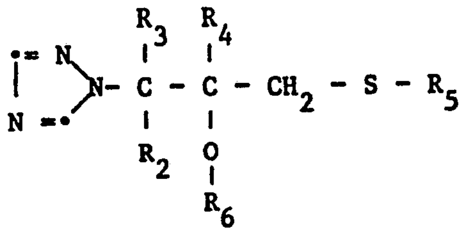

| Nr. | $R_2$ | $R_3$ | $R_4$ | $R_5$ | $R_6$ | phys. Daten |
|---|---|---|---|---|---|---|
| 201 | $CH_3$ | H | t-$C_4H_9$ | 4-Cl-$C_6H_4$- | H | hochviskoses Öl |
| 202 | $CH_3$ | H | t-$C_4H_9$ | 4-F-$C_6H_4$- | H | |
| 203 | $CH_3$ | H | t-$C_4H_9$ | 4-Br-$C_6H_4$- | H | |
| 204 | $C_2H_5$ | H | t-$C_4H_9$ | 4-Cl-$C_6H_4$- | H | |
| 205 | $C_2H_5$ | H | t-$C_4H_9$ | 4-F-$C_6H_4$- | H | |
| 206 | $CH_3$ | H | i-$C_3H_7$ | 4-F-$C_6H_4$- | H | |
| 207 | $CH_3$ | H | i-$C_3H_7$ | 4-F-$C_6H_4$- | $CH_3$ | |
| 208 | $CH_3$ | H | t-$C_4H_9$ | 4-F-$C_6H_4$- | $CH_3$ | |
| 209 | $CH_3$ | H | t-$C_4H_9$ | 4-Cl-$C_6H_4$- | $CH_3$ | |
| 210 | $C_2H_5$ | H | t-$C_4H_9$ | 4-Cl-$C_6H_4$- | $CH_3$ | |
| 211 | $C_2H_5$ | H | t-$C_4H_9$ | 4-F-$C_6H_4$- | $CH_3$ | |
| 212 | $CH_3$ | H | t-$C_4H_9$ | n-$C_4H_9$- | H | Öl |
| 213 | $CH_3$ | H | t-$C_4H_9$ | $C_6H_5$-$CH_2$- | H | |

**Tabelle 3:**

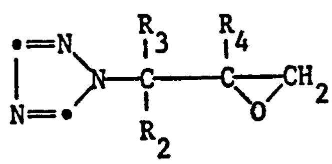

| Nr. | $R_2$ | $R_3$ | $R_4$ | $R_5$ | $R_6$ | X | phys. Daten |
|---|---|---|---|---|---|---|---|
| 301 | $CH_3$ | H | t-$C_4H_9$ | 4-Cl-$C_6H_4$- | H | O | Smp. 121-123° C |
| 302 | $CH_3$ | H | t-$C_4H_9$ | 4-Cl-$C_6H_4$- | H | S | |
| 303 | $CH_3$ | H | t-$C_4H_9$ | 4-Br-$C_6H_4$- | H | O | Smp. 130-132° C |
| 304 | $CH_3$ | H | t-$C_4H_9$ | 4-Br-$C_6H_4$- | H | S | |
| 305 | $CH_3$ | H | t-$C_4H_9$ | 4-$CH_3$-$C_6H_4$- | H | O | Smp. 129-130° C |
| 306 | $CH_3$ | H | t-$C_6H_9$ | 4-$CH_3$-$C_6H_4$- | H | S | |
| 307 | $CH_3$ | H | t-$C_4H_9$ | $C_6H_5$ | H | O | hochviskoses Öl |
| 308 | $CH_3$ | H | t-$C_4H_9$ | $C_6H_5$ | H | S | |
| 309 | $CH_3$ | H | t-$C_4H_9$ | 4-F-$C_6H_4$- | H | O | |
| 310 | $CH_3$ | H | t-$C_4H_9$ | 4-F-$C_6H_4$- | H | S | |
| 311 | $CH_3$ | H | t-$C_4H_9$ | 4-F-$C_6H_4$ | $CH_3$ | O | |
| 312 | $CH_3$ | $CH_3$ | t-$C_4H_9$ | 4-F-$C_6H_4$- | H | O | |
| 313 | $CH_3$ | $CH_3$ | t-$C_4H_9$ | 4-F-$C_6H_4$- | $CH_3$ | O | |
| 314 | $CH_3$ | $CH_3$ | t-$C_4H_9$ | 4-F-$C_6H_4$- | $CH_3$ | S | |
| 315 | $CH_3$ | H | t-$C_4H_9$ | 4-F-$C_6H_4$- | $CH_3$ | S | |

**Tabelle 4:**

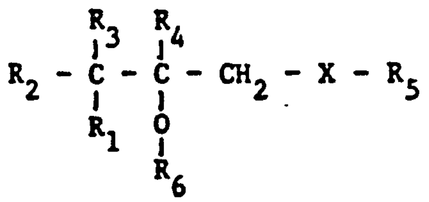

(I).

| Nr. | $R_2$ | $R_3$ | $R_4$ | phys. Daten |
|---|---|---|---|---|
| 401 | $CH_3$ | H | $t\text{-}C_4H_9$ | Sdp. 68-70° C, 0,01 mb |
| 402 | $C_2H_5$ | H | $t\text{-}C_4H_9$ | Sdp. 70-72° C, 0,1 mb |
| 403 | $n\text{-}C_3H_7$ | H | $t\text{-}C_4H_9$ | Sdp. 80-82° C, 0,05 mb |
| 404 | $C_2H_5$ | $CH_3$ | $t\text{-}C_4H_9$ | |
| 405 | $CH_3$ | $CH_3$ | $t\text{-}C_4H_9$ | Öl |
| 406 | $n\text{-}C_4H_9$ | H | $t\text{-}C_4H_9$ | Sdp. 80° C, 0,05 mb |
| 407 | $CH_3$ | H | $-C(CH_3)_2\text{-}C_2H_5$ | |
| 408 | $CH_3$ | H | $i\text{-}C_3H_7$ | Öl |
| 409 | $CH_3$ | H | $n\text{-}C_6H_{13}$ | Öl |
| 410 | $CH_3$ | H | $i\text{-}C_5H_{11}$ | |

**Formulierungsbeispiele**

**Beispiel 5:**

Formulierungsbeispiele für flüssige Wirkstoffe der Formel I (% = Gewichtsprozent)

| a) Emulsionskonzentrate | a) | b) | c) |
|---|---|---|---|
| Wirkstoff | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenoyl-polyäthylenglykoläther (30 Mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) | d) |
|---|---|---|---|---|
| Wirkstoff | 80 % | 10 % | 5 % | 95 % |
| Äthylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190° C) | - | - | 94 % | - |

Die Lösungen sind zur Anwendung in Form kleinster Tropfen geeignet.

c) Granulate

| | a) | b) |
|---|---|---|
| Wirkstoff | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

d) Stäubemittel

| | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

**Beispiel 6:**

Formulierungsbeispiele für feste Wirkstoffe der Formel I (% = Gewichtsprozent)

a) Spritzpulver

| | a) | b) |
|---|---|---|
| Wirkstoff | 20 % | 60 % |
| Na-Ligninsulfonat | 5 % | 5 % |
| Na-Laurylsulfat | 3 % | - |
| Na-Diisobutylnaphthalinsulfonat | - | 6 % |
| Octylphenolpolyäthylenglykoläther (7-8 Mol AeO) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | |
|---|---|
| Wirkstoff | 10 % |
| Octylphenolpolyäthylenglykoläther (4-5 Mol ÄO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

d) Extruder Granulat

| | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | |
|---|---|
| Wirkstoff | 40 % |
| Äthylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Biologische Beispiele**

**Beispiel 7: Wirkung gegen Puccinia graminis auf Weizen**

a) Residual-protektive Wirkung
Weizenpflanzen werden 6 Tage nach der Aussaat mit einer aus Spritzpulver des Wirkstoffes hergestellten Spritzbrühe (0,06 % Aktivsubstanz) besprüht. Nach 24 Stunden werden die behandelten Pflanzen mit einer Ureidosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rotpustelentwicklung erfolgt 12 Tage nach der Infektion.
b) Systemische Wirkung
Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Ureidosporensuspension des Pilzes infiziert. Nach einer Inkubation während 48 Stunden bei 95-100 % relativer Luftfeuchtigkeit und ca. 20° C werden die infizierten Pflanzen in einem Gewächshaus bei ca. 22° C aufgestellt. Die Beurteilung der Rostpustelentwicklung erfolgt 12 Tage nach der Infektion.
Verbindungen aus der Tabelle I zeigen gegen Puccinia-Pilze eine gute Wirkung. Unbehandelte aber infizierte Kontrollpflanzen zeigen einen Puccinia-Befall von 100 %. Unter anderem hemmen die Verbindungen 1 bis 3 13, 14, 15, 27, 28, 67, 70, 73, 79, 103 und 105 den Puccinia-Befall auf 0 bis 5 %.

**Beispiel 8: Wirkung gegen Cercospora arachidicola auf Erdnusspflanzen**

a) Residual-protektive Wirkung
10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21° C und hoher Luftfeuchtigkeit inkubiert

und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

b) Systemische Wirkung

Zu 10-15 cm hohen Erdnusspflanzen wird eine aus Spritzpulver des Wirkstoffes hergestellte Spritzbrühe gegossen (0,06 % Aktivsubstanz bezogen auf das Erdvolumen). Nach 48 Stunden werden die behandelten Pflanzen mit einer Konidiensuspension des Pilzes infiziert und 72 Stunden bei ca. 21°C und hoher Luftfeuchtigkeit inkubiert. Anschliessend werden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken = 100 %), zeigen Erdnusspflanzen, die mit Wirkstoffen aus der Tabelle I behandelt werden, einen stark reduzierten Cercospora-Befall. So verhindern die Verbindungen 1 bis 3, 13, 14, 15, 27, 28, 67, 73, 79, 103, und 105 in obigen Versuchen das Auftreten von Flecken fast vollständig (0-10 %).

**Beispiel 9: Wirkung gegen Erysiphae graminis auf Gerste**

a) Residual-protektive Wirkung

Ca. 8 cm hohe Gerstenpflanzen werden mit einer aus Spritzpulver des Wirkstoffes hergestellten Spritzbrühe (0,02 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit Konidien des Pilzes bestäubt. Die infizierten Gerstenpflanzen werden in einem Gewächshaus bei ca. 22°C aufgestellt und der Pilzebefall nach 10 Tagen beurteilt.

Verbindungen der Formel I zeigen eine gute Wirkung gegen Erysiphe-Pilze. Unbehandelte, aber infizierte Kontrollpflanzen zeigen einen Erysiphe-Befall von 100 %. Unter anderen Verbindungen aus der Tabelle I hemmen die Verbindungen Nr. 1 bis 3, 13, 14, 15, 27, 28, 73, 79, 103 und 105 bis 107 den Pilzbefall bei Gerste auf 0 bis 5 %.

**Beispiel 10: Residual-protektive Wirkung gegen Venturia inaequalis auf Apfeltrieben**

Apfelstecklinge mit 10-20 cm langen Frischtrieben werden mit einer aus Spritzpulver des Wirkstoffes hergestellten Spritzbrühe (0,06 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt. Verbindungen 1 bis 3, 13, 14, 15, 27, 28, 67, 73, 79, 105 und andere hemmen den Krankheitsbefall auf weniger als 10 %. Unbehandelte aber infizierte Triebe zeigen einen 100 %igen Venturia-Befall.

**Beispiel 11: Wirkung gegen Botrytis cinerea auf Bohnen**

Residual protektive Wirkung

Ca. 10 cm hohe Bohnen-Pflanzen werden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21°C erfolgt die Beurteilung des Pilzbefalles. Die Verbindungen aus der Tabelle I hemmen in vielen Fällen die Pilzinfektion sehr stark. Bei einer Konzentration von 0,02 % erweisen sich z. B. die Verbindungen Nr. 1 103 und 103 bis 107 als voll wirksam (Krankheitsbefall 0 bis 5 %).

Der Botrytis-Befall ungehandelter aber infizierter Bohnenpflanzen betrug 100 %.

**Beispiel 12: Wuchshemmung bei Getreide**

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21

Tage nach der Aussaat mit der wässrigen Spritzbrühe eines Wirkstoffes der Formel 1 besprüht. Die Wirkstoffmenge beträgt umgerechnet 4 bzw. 2,5 kg Aktivsubstanz pro Hektar. 10 und 21 Tage nach Applikation wird das Wachstum des Getreides beurteilt. Hierbei kann festgestellt werden, dass Getreidepflanzen, die mit Wirkstoffen der Formel I behandelt worden sind, im Vergleich zu unbehandelten Kontrollpflanzen eine starke Wuchsreduktion aufweisen.

| Verb.Nr. | Aufwandmenge AS kg/ha | Pflanzengrösse in % zur Kontrollpflanze (Kontrolle = 100 %) | |
|---|---|---|---|
| | | Getreide | |
| | | Gerste | Roggen |
| 1 | 4 | 27 | - |
| 4 | 2,5 | 75 | 46 |
| 13 | 2,5 | 14 | 7 |
| 14 | 2,5 | 21 | 11 |
| 16 | 2,5 | 42 | 31 |
| 27 | 2,5 | 56 | 40 |
| 28 | 2,5 | 88 | 81 |
| 32 | 2,5 | 81 | 73 |
| 45 | 2,5 | 69 | 24 |
| 47 | 2,5 | 72 | 80 |
| 50 | 2,5 | 26 | 30 |
| 52 | 2,5 | 74 | 78 |
| 58 | 2,5 | 37 | 18 |
| 73 | 2,5 | 81 | 81 |
| 93 | 2,5 | 66 | 92 |
| 108 | 2,5 | 50 | 37 |
| 109 | 2,5 | 60 | 55 |
| 110 | 2,5 | 45 | 60 |
| 111 | 2,5 | 47 | 45 |
| 112 | 2,5 | 37 | 40 |

**Beispiel 13: Wuchshemmung bei Gräsern**

In Kunststoffschalen mit Erde-Torf-Sand-Gemisch (6:3:1) wird ein Grasgemisch enthaltend Poa pratensis, Dactylis glomerata, Lolium perenne, Festuca rubra, Festuca ovina, Cynosurus crystatus, Agropyron repens und Bromus inermis im Gewächshaus angesät und nach Bedarf bewässert. Die aufgelaufenen Gräser werden wöchentlich bis auf ca. 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet 0,5 bzw. 2,5 kg Aktivsubstanz pro Hektar. 10 und 21 Tage nach der Applikation wird das Durchschnitts-Wachstum der Gräser beurteilt, dabei zeigt es sich, dass die erfindungsgemässen Wirkstoffe auf den Tabellen I bis 3 eine merkliche Wuchshemmung bewirken.

| Verb.-Nr. | Aufwandmenge AS kg/ha | Pflanzengrösse in % zur Kontrollpflanze (Kontrolle = 100 %) |
|---|---|---|
| | | Gras-Mischung |
| 1 | 4 | 40 |
| 4 | 2,5 | 47 |
| 13 | 2,5 | 23 |
| 14 | 2,5 | 32 |
| 16 | 2,5 | 38 |
| 27 | 2,5 | 50 |
| 28 | 2,5 | 82 |
| 32 | 2,5 | 76 |
| 45 | 2,5 | 32 |
| 47 | 2,5 | 90 |
| 50 | 2,5 | 55 |
| 52 | 2,5 | 86 |
| 58 | 2,5 | 48 |
| 73 | 2,5 | 76 |
| 93 | 2,5 | 81 |
| 108 | 2,5 | 44 |
| 109 | 2,5 | 44 |
| 110 | 2,5 | 41 |
| 111 | 2,5 | 48 |
| 112 | 2,5 | 38 |

**Beispiel 14: Ertragssteigerung en Sojabohnen**

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffes der Formel I bis zur guten Benetzung besprüht. Die Wirkstoffkonzentration beträgt umgerechnet bis zu 3 kg Aktivsubstanz pro Hektar. Die Auswertung erfolgt ca. 5 Wochen nach der Applikation des Wirkstoffs. Im Vergleich zu unbehandelten Kontrollplanzen bewirken die erfindungsgemässen Wirkstoffe der Formel I eine merkliche Erhöhung der Anzahl und des Gewichts der geernteten Schoten.

| Verb.Nr | Anzahl Schoten in % zur Kontrollpflanze (Kontrolle = 100 %) | Gesamtgewicht der Schoten in % zur Kontrollpflanze (Kontrolle = 100 %) |
|---|---|---|
| 1 | 98 | 103 |
| 13 | 105 | 108 |
| 14 | 107 | 111 |
| 27 | 100 | 104 |
| 108 | 107 | 107 |

**Beispiel 15: Hemmung des Vegetativ-Wachstums an Soja**

In Kunststofftöpfen mit einem Erde-Torf-Sandgemisch im Verhältnis 6:3:1 werden die Sojabohnen der Sorte "Hark" angesät, im Gewächshaus aufgestellt und nach Bedarf bewässert. 15 Tage nach der Saat werden die Pflanzen mit der wässrigen Spritzbrühe eines Wirkstoffes der Formel I bis zur Benetzung besprüht. Die Wirkstoffkombination beträgt umgerechnet 1-1,5 kg Aktivsubstanz pro Hektar. 7 und 14 Tage nach der Applikation wird das Wachstum der Pflanzen beurteilt. Dabei zeigt sich dass die erfindungsgemässen Wirkstoffe aus den Tabellen 1-3 eine merkliche Wuchshemmung bewirken.

| Verb.-Nr. | Aufwandmenge AS kg/ha | Pflanzengrösse in % zur Kontrollpflanze (Kontrolle = 100 %) |
|---|---|---|
| 1 | 1 | 9 |
| 4 | 1,5 | 13 |
| 13 | 1,5 | 13 |
| 14 | 1,5 | 31 |
| 16 | 1,5 | 15 |
| 27 | 1,5 | 35 |
| 28 | 1,5 | 31 |
| 32 | 1,5 | 23 |
| 45 | 1,5 | 13 |
| 46 | 1,5 | 87 |
| 50 | 1,5 | 82 |
| 52 | 1,5 | 93 |
| 53 | 1,5 | 87 |
| 54 | 1,5 | 36 |
| 58 | 1,5 | 71 |
| 68 | 1,5 | 23 |
| 73 | 1,5 | 77 |
| 77 | 1,5 | 31 |
| 79 | 1,5 | 77 |
| 108 | 1,5 | 62 |
| 109 | 1,5 | 23 |
| 110 | 1,5 | 27 |
| 111 | 1,5 | 73 |
| 112 | 1,5 | 78 |
| 113 | 1,5 | 100 |

**Beispiel 16: Wuchshemmung bei Bodenbedecker-Pflanzen (Cover Crops)**

In Kunststoffschalen mit Erde-Torf-Sand-Gemisch (1:1:1) werden Testpflanzen der Sorte Psophocarpus palustris und Centrosema pubescens aus Stecklingen angezogen. Nach dem Anwurzeln werden die Pflänzchen in 9-cm-Töpfe umgetopft und nach Bedarf gewässert. Die weitere Anzucht der Pflanzen findet im Gewächshaus bei einer Tagestemperatur von 27° C und einer Nachttemperatur von 21° C, bei einer mittleren Lichtdauer von 14 h (6000 Lux) und einer Luftfeuchtigkeit von 70 % statt. Die Testpflanzen werden auf eine Höhe von ca. 15 cm zurückgeschnitten und 5 Tage nach dem Zurückschneiden mit einer Spritzbrühe des Wirkstoffes (umgerechnet 1 bzw. 3 kg Aktivsubstanz je Hektar) besprüht. 4 Wochen nach Applikation wird das Wachstum der behandelten Pflanzen im Vergleich zu gestutzten, aber unbehandelten Kontrollpflanzen verglichen. Hierbei kann festgestellt werden, dass Verbindungen aus den Tabellen 1 bis 3 eine deutliche Wuchshemmung der Bodenbedecker-Pflanzen auslösen.

| Verb.-Nr. | Aufwandmenge AS kg/ha | Pflanzengrösse in % zur Kontrollpflanze (Kontrolle = 100 %) | |
|---|---|---|---|
| | | Centrosema | Psophocarpus |
| 1 | 3 | 10 | 10 |
| 13 | 3 | 10 | 10 |
| 14 | 3 | 40 | 80 |
| 16 | 1 | 10 | 10 |
| 27 | 3 | 10 | 10 |
| 28 | 1 | 10 | 30 |
| 45 | 3 | 60 | 20 |
| 70 | 1 | 60 | 50 |
| 108 | 3 | 90 | 70 |
| 110 | 3 | 40 | 40 |

**Beispiel 17: Wuchsterminierung Baumwolle**

In Kunststoffbehältern mit einem Erde-Torf-Gemisch im Verhältnis von 2:1 werden Baumwollpflanzen der Sorte Delta Pine angesät und im Gewächshaus bei Temperaturen von 20°-26° C angezogen. Nach 2 Monaten haben sich die Pflanzen bis zum 6 Blattstadium entwickelt. Zu diesem Zeitpunkt werden die Pflanzen mit einer wässrigen Dispersion eines Wirkstoffes der Formel I bis zur guten Benetzung besprüht. Die

Wirkstoffkonzentration beträgt umgerechnet 2,0 kg Aktivsubstanz pro Hektar. Die Auswertung erfolgt ca. 1 Monat nach der Applikation des Wirkstoffs. Im Vergleich zu unbehandelten Kontrollpflanzen bewirken die erfindungsgemässen Wirkstoffe der Formel I eine merkliche Reduktion des Neuzuwachses.

| Verb.-Nr. | Aufwandmenge AS kg/ha | Neuzuwachs in % zur Kontrollpflanze (Kontrolle = 100 %) |
|---|---|---|
| 14 | 2 | 17 |
| 16 | 2 | 17 |
| 32 | 2 | 14 |

**Patentansprüche** für die Vertragsstaaten BE, CH, DE, FR, GB, IT, LI, NL, SE.

1. Azolylpropan-Deriwate der allgemeinen Formel I

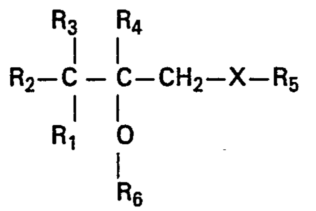

(I)

worin

$R_1$ für 1H-1,2,4,-Triazol-1-yl oder 1H'-Imidazol-1-yl steht,

$R_2$ $C_1$-$C_4$-alkyl und $R_3$ Wasserstoff, $C_1$-$C_4$-Alkyl oder gegebenenfalls einfach durch Halogen substituiertes Benzyl bedeuten,

$R_4$ für $C_1$-$C_6$-Alkyl oder für gegebenenfalls einfach durch Halogen, Cyan, Nitro, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy oder $C_1$-$C_4$-Halogenalkylthio substituiertes Phenyl steht,

$R_5$ für $C_1$-$C_8$-Alkyl, $C_3$-$C_8$-Cycloalkyl, Phenylalkyl mit 1-4 Kohlenstoffatomen im Alkylteil oder gegebenenfalls ein- oder mehrfach durch Halogen, Cyan, Nitro, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy oder $C_1$-$C_4$-Halogenalkylthio substituiertes Phenyl steht,

$R_6$ Wasserstoff, $C_1$-$C_4$-Alkyl, Phenylalkyl mit 1-4 Kohlenstoffatomen im Alkylteil, $C_1$-$C_6$-Alkylcarbonyl, Carbamoyl, an dessen Sticksoffatom unabhängig von einander Wasserstoff, $C_1$-$C_4$-Alkoxy oder $C_1$-$C_4$-Alkyl gebunden sind, $C_1$-$C_4$-Alkoxycarbonyl, $C_1$-$C_4$-Alkylsulfonyl, gegebenenfalls einfach durch $C_1$-$C_4$-Alkyl substituiertes Phenylsulfonyl oder Sulfamoyl an dessen Stickstoffatom unabhängig von einander Wasserstoff oder $C_1$-$C_4$-Alkyl gebunden sind, und

X Sauerstoff oder Schwefel bedeuten, unter Einschluss der Säureadditionssalze der quaternären Azoliumsalze und der Metallkomplexe

2. Verbindungen gemäss Anspruch 1 dadurch gekennzeichnet, dass $R_1$ für 1H-1,2,4-Triazol-1-yl steht.

3. Verbindungen gemäss Ansprüch 1, dadurch gekennzeichnet, dass $R_4$ für tert.-Butyl steht.

4. Verbindungen gemäss Anspruch 1, dadurch gekennzeichnet, dass $R_6$ Wasserstoff, Methyl, Benzyl, Acetyl, Methylcarbamoyl, N,N-Dimethylcarbamoyl N-Methoxy-N-methylcarbamoyl, Methoxycarbonyl, Äthoxycarbonyl, Methylsulfonyl, 4-Tolylsulfonyl, Methylsulfamoyl oder N,N-Dimethylsulfamoyl bedeutet.

5. Verbindungen gemäss Anspruch 1, dadurch gekennzeichnet, dass $R_1$ für 1H-1,2,4-Triazol-1-yl steht, $R_2$ $C_1$-$C_4$-Alkyl und $R_3$ Wasserstoff oder $C_1$-$C_4$-Alkyl bedeuten, $R_4$ für $C_1$-$C_6$-Alkyl oder für gegebenenfalls einfach durch Halogen, Cyan, Nitro, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy oder $C_1$-$C_4$-Halogenalkylthio substituiertes Phenyl steht, $R_5$ für $C_1$-$C_8$-Alkyl, $C_3$-$C_8$-Cycloalkyl, Phenylalkyl mit 1-4 Kohlenstoffatomen im Alkylteil oder gegebenenfalls ein- oder mehrfach durch Halogen, Cyan, Nitro, $C_1$-$C_4$-Alkyl, $C_1$-$C_4$-Alkoxy, $C_1$-$C_4$-Alkylthio, $C_1$-$C_4$-Halogenalkyl $C_1$-$C_4$-Halogenalkoxy oder $C_1$-$C_4$-Halogenalkylthio substituiertes Phenyl steht und $R_6$ Wasserstoff, Methyl, Benzyl, Acetyl, Methylcarbamoyl, N,N-Dimethylcarbamoyl, N-Methoxy-N-methylcarbamoyl, Methoxycarbonyl, Äthoxycarbonyl, Methylsulfonyl, 4-Tolylsulfonyl, Methylsulfamoyl oder N,N-Dimethylsulfamoyl bedeutet.

6. Verbindungen gemäss Anspruch 1, dadurch gekennzeichnet, dass $R_1$ für 1H-1,2,4-Triazol-1-yl, $R_2$ für Methyl oder Äthyl, $R_3$ Wasserstoff, $R_4$ für iso-Propyl oder tert.Butyl, $R_5$ für unsubstituiertes oder ein- bis zweimal durch Methyl, Nitro, Fluor, Chlor oder Brom substituiertes Phenyl, X für Sauerstoff und $R_6$ für Wasserstoff oder Methyl stehen.

7. 1-(4-Chlorphenoxy)-2-tert.butyl-2-hydroxy-3-methyl-3-(1H-1,2,4-triazol-1-yl)-propan gemäss Anspruch 1.

8. 1-(4-Toluyloxy)-2-tert.butyl-2-hydroxy-3-methyl-3-(1H-1,2,4-triazol-1-yl)-propan gemäss Anspruch 1.

9. 1-(4-Chlorphenoxy)-2-tert.butyl-2-hydroxy-3-äthyl-3-(1H-1,2,4-triazol-1-yl)-propan gemäss Anspruch 1.

10. 1-(4-Fluorphenoxy)-2-tert.butyl-2-hydroxy-3-methyl-3-(1H-1,2,4-triazol-1-yl)-propan gemäss Anspruch 1.

11. 1-Phenoxy-2-tert.butyl-2-methoxy-3-methyl-3-(1H-1,2,4-triazol-1-yl)-propan gemäss Anspruch 1.

12. 1-(4-Fluorphenoxy)-2-tert.butyl-2-hydroxy-3-äthyl-3-(1H-1,2,4-triazol-1-yl)-propan gemäss Anspruch 1.

13. 1-(2-Fluorphenoxy)-2-tert.butyl-2-hydroxy-3-methyl-(1H-1,2,4-triazol-1-yl)-propan gemäss Anspruch 1.

14. 1-(4-Bromphenoxy)-2-tert.butyl-2-hydroxy-3-methyl-3-(1H-1,2,4-triazol-1-yl)-propan gemäss Anspruch 1.

15. 1-(4-Chlor-2-methylphenoxy)-2-tert.butyl-2-hydroxy-3-methyl-3-(1H-1,2,4-triazol-1-yl)-propan gemäss Anspruch 1.

16 1-(4-Fluorphenoxy)-2-isopropyl-2-hydroxy-3-methyl-3-(1H-1,2,4-triazol-1-yl)-propan gemäss Anspruch 1.

17. Azolylmethyloxirane der allgemeinen Formel II

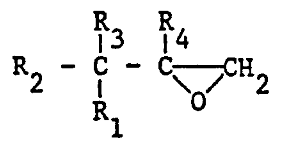

(II),

worin $R_1$, $R_2$, $R_3$ und $R_4$ die unter Formel I, im Anspruch 1, gegebene Bedeutung haben.

18. Verfahren zur Herstellung der Verbindungen der Formel I, dadurch gekennzeichnet, dass man ein Azolylmethyloxiran der Formel II

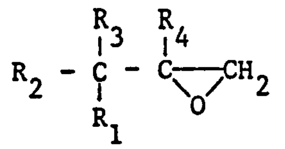

(II)

worin $R_1$, $R_2$, $R_3$ und $R_4$ die unter Formel I gegebene Bedeutung haben, in Gegenwart einer Base in einem inerten Lösungsmittel mit einem Alkohol oder Thioalkohol der Formel III

$$H-X-R_5 \qquad (III),$$

worin X und $R_5$ die unter Formel I gegebene Bedeutung hat, umsetzt und das Produkt der Formel Ia

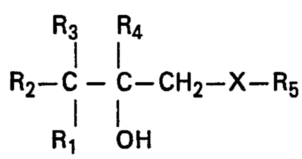

(Ia)

gewünschtenfalls durch Umsetzung mit einem Verätherungs- oder Veresterungsmittel der Formel IV

$$Y-R_6 \qquad (IV),$$

worin $R_6$ die unter Formel I gegebene Bedeutung hat und Y ein Halogenatom oder einen organischen oder anorganischen Säurerest bedeutet, veräthert oder verestert.

19. Mittel zur Bekämpfung oder Verhütung eines Befalls durch Mikroorganismen und/oder zur Regulierung des Pflanzenwachstums, dadurch gekennzeichnet, dass es neben Träger- und/oder anderen Zuschlagstoffen als Wirkstoff mindestens eine Verbindung der Formel I gemäss Anspruch 1 enthält.

20. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine gemäss Anspruch 1 definierte Verbindung der Formel I in einer wirksamen Menge auf die Pflanze oder deren Standort appliziert.

21. Verwendung von Verbindungen der Formel I gemäss Anspruch 1 zur Bekämpfung und/oder zur präventiven Verhütung eines Befalls von Mikroorganismen und/oder zur Regulierung des Pflanzenwachstums.

22. Verfahren gemäss Anspruch 20, dadurch gekennzeichnet, dass es sich bei den Mikroorganismen um phytopathogene Pilze handelt.

23. Verfahren gemäss Anspruch 20 zur Wuchshemmung im Sinne einer Erhöhung der Knickfestigkeit und Halmverkürzung bei den Getreidesorten Hafer, Weizen, Gerste oder Roggen.

24. Verfahren gemäss Anspruch 20 zur Wuchshemmung bei Gräsern und Unkräutern.

25. Verfahren gemäss Anspruch 20 zur Wuchsregulierung bei Soja in Sinne einer Ertragssteigerung.

26. Verfahren gemäss Anspruch 20 zur Wuchshemmung von Bodenbedeckerpflanzen.

27. Verfahren gemäss Anspruch 20 zur Wuchshemmung und zur Wuchsregulierung von Baumwollpflanzen im Sinne einer Ertragssteigerung.

28. Verfahren gemäss Anspruch 20 zur Wuchsregulierung bei Getreidepflanzen im Sinne einer Ertragssteigerung.

29. Verfahren gemäss Anspruch 20 zur Seneszenzhemmung von Pflanzen im Sinne einer Ertragssteigerung.

**Patentansprüche** für den Vertragsstaat AT.

1. Mittel zur Bekämpfung oder Verhütung eines Befalls durch Microorganismen und/oder zur Regulierung des Pflanzenwachstums, dadurch gekennzeichnet, dass es neben Träger- und/oder anderen Zuschlagstoffen als Wirkstoff mindestens ein Azolylpropan-Derivat der allgeneinen Formel I

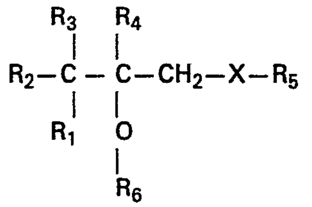

(I)

enthält, worin

$R_1$ für 1H-1,2,4-Triazol-1-yl oder 1H'-Imidazol-1-yl

$R_2$ $C_1$-$C_4$-Alkyl und $R_3$ Wasserstoff, $C_1$-$C_4$-Alkyl oder gegebenenfalls einfach durch Halogen substituiertes Benzyl bedeuten,

$R_4$ für $C_1$-$C_6$-Alkyl oder für gegebenenfalls einfach durch Halogen, Cyan, Nitro, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy oder $C_1$-$C_4$-Halogenalkylthio substituiertes Phenyl steht,

$R_5$ für $C_1$-$C_8$-Alkyl, $C_3$-$C_8$-Cycloalkyl, Phenylalkyl mit 1-4 Kohlenstoffatomen im Alkylteil oder gegebenenfalls ein- oder mehrfach, durch Halogen, Cyan, Nitro, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy oder $C_1$-$C_4$-Halogenalkylthio substituiertes Phenyl steht,

$R_6$ Wasserstoff, $C_1$-$R_4$-Alkyl, Phenylalkyl mit 1-4 Kohlenstoffatomen im Alkylteil, $C_1$-$C_6$-Alkylcarbonyl, Carbamoyl, an dessen Stickstoffatomen unabhänging von einander Wasserstoff, $C_1$-$C_4$-Alkoxy oder $C_1$-$C_4$-Alkyl gebunden sind,

$C_1$-$C_4$-Alkoxycarbonyl, $C_1$-$C_4$-Alkylsulfonyl, gegebenenfalls einfach durch $C_1$-$C_4$-Alkyl substituiertes phenylsulfonyl oder Sulfamoyl, an dessen Stickstoffatom unabhängig von einander Wasserstoff oder $C_1$-$C_4$-Alkyl gebunden sind, und

X Sauerstoff oder Schwefel bedeutet unter Einschluss der Säureadditionssalze der quaternären Azoliumsalze und der Metallkomplexe.

2. Mittel gemäss Anspruch 1, dadurch gekennzeichnet, dass $R_1$ für 1H-1,2,4-Triazol-1-yl steht.

3. Mittel gemäss Anspruch 1, dadurch gekennzeichnet, dass $R_4$ für tert.-Butyl steht.

4. Mittel gemäss Anspruch 1, dadurch gekennzeichnet, dass $R_6$ Wasserstoff, Methyl, Benzyl, Acetyl, Methylcarbamoyl, N,N-Dimethylcarbamoyl, N-Methoxy-N-methylcarbamoyl, Methoxycarbonyl, Äthoxycarbonyl, Methylsulfonyl, 4-Tolylsulfonyl, Methylsulfamoyl oder N,N-Dimethylsulfamoyl bedeutet.

5. Mittel gemäss Anspruch 1, dadurch gekennzeichnet, dass $R_1$ für 1H-1,2,4-Triazol-1-yl steht, $R_2$ $C_1$-$C_4$-Alkyl und $R_3$ Wasserstoff oder $C_1$-$C_4$-Alkyl bedeuten, $R_4$ für $C_1$-$C_6$-Alkyl oder für gegebenenfalls einfach durch Halogen, Cyan, Nitro, $C_1$-$C_4$-Alkyl $C_1$-$C_4$-Alkylthio, $C_1$-$C_4$-Halogenalkyl, $C_1$-$C_4$-Halogenalkoxy oder $C_1$-$C_4$-Halogenalkylthio substituiertes Phenyl steht, $R_5$ für $C_1$-$C_8$-Alkyl, $C_3$-$C_8$-Cycloalkyl, Phenylalkyl mit 1-4 Kohlenstoffatomen im Alkylteil oder gegebenenfall ein- oder mehrfach durch Halogen, Cyan, Nitro, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy oder $C_1$-$C_4$-Halogenalkylthio substituiertes Phenyl steht und $R_6$ Wasserstoff, Methyl, Benzyl, Acetyl, Methylcarbamoyl, N,N-Dimethylcarbamoyl, N-Methoxy-N-methylcarbamoyl, Methoxycarbonyl, Äthoxycarbonyl, Methylsulfonyl, 4-Tolylsulfonyl, Methylsulfamoyl oder N,N-Dimethylsulfamoyl bedeutet.

6. Mittel gemäss Anspruch 1, dadurch gekennzeichnet, dass $R_1$ für 1H-1,2,4-Triazol-1-yl, $R_2$ für Methyl oder Äthyl, $R_3$ Wasserstoff, $R_4$ für iso-Propyl oder tert. Butyl, $R_5$ für unsubstituiertes oder ein- bis zweimal durch Methyl, Nitro, Fluor, Chlor oder Brom substituiertes Phenyl, X für Sauerstoff und $R_6$ für Wasserstoff oder Methyl stehen.

7. Mittel gemäss Anspruch 1, dadurch gekennzeichnet, dass es als Wirkstoff eine Verbindung enthält ausgewählt aus der Gruppe 1-(4-Chlorphenoxy)-2-tert.butyl-2-hydroxy-3-methyl-3-(1H-1,2,4-triazol-1-yl)-propan, 1-(4-Toluyloxy)-2-tert.butyl-2-hydroxy-3-methyl-3-(1H-1,2,4-triazol-1-yl)-propan, 1-(4-Chlorphenoxy)-2-tert.butyl-2-hydroxy-3-äthyl-3-(1H-1,2,4-triazolyl-1-yl)-propan, 1-(4-Fluorphenoxy)-2-tert.butyl-2-hydroxy-3-methyl-3-(1H-1,2,4-triazol-1-yl)-propan, 1-Phenoxy-2-tert.butyl-2-methoxy-3-methyl-3-(1H-1,2,4-triazol-1-yl)-propan, 1-(4-Fluorphenoxy)-2-tert.butyl-2-hydroxy-3-äthyl-3-(1H-1,2,4-triazol-1-yl)-propan, 1-(2-Fluorphenoxy)-2-tert.butyl-2-hydroxy-3-methyl-(1H-1,2,4-triazol-1-yl)-propan, 1-(4-Bromphenoxy)-2-tert.butyl-2-hydroxy-3-methyl-3-(1H-1,2,4-triazol-1-yl)-propan, 1-(4-Chlor-2-methylphenoxy)-2-tert.butyl-2-hydroxy-3-methyl-3-(1H-1,2,4-triazol-1-yl)-propan oder 1-(4-Fluorphenoxy)-2-isopropyl-2-hydroxy-3-methyl-3-(1H-1,2,4-triazol-1-yl)-propan.

8. Verfahren zur Herstellung der Verbindungen der Formel I, dadurch gekennzeichnet, dass man ein Azolylmethyloxiran der Formel II

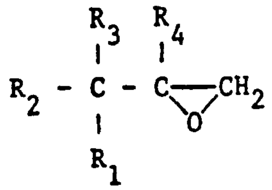

(II)

worin $R_1$, $R_2$, $R_3$ und $R_4$ die unter Formel I gegebene Bedeutung haben in Gegenwart einer Base in einem inerten Lösungsmittel mit einem Alkohol oder Thioalkohol der Formel III

$$H-X-R_5 \quad \text{(III)},$$

worin X und $R_5$ die unter Formel I gegebene Bedeutung hat, umsetzt und das Produkt der Formel Ia

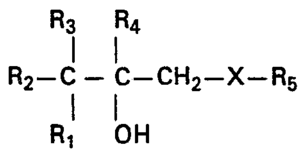

(Ia)

gewünschtenfalls durch Umsetzung mit einem Verätherungs- oder Veresterungsmittel der Formel IV

$$Y-R_6 \quad \text{(IV)},$$

worin $R_6$ die unter Formel I gegebene Bedeutung hat und Y ein Halogenatom oder einen organischen oder anorganischen Säurerest bedeutet, veräthert oder verestert.

9. Verfahren zur Bekämpfung oder Verhütung eines Befalls von Kulturpflanzen durch phytopathogene Mikroorganismen und/oder zur Regulierung des Pflanzenwachstums, dadurch gekennzeichnet, dass man eine gemäss Anspruch 1 definierte Verbindung der Formel I in einer wirksamen Menge auf die Pflanze oder deren Standort appliziert.

10. Verwendung von Verbindungen der Formel I gemäss Anspruch 1 zur Bekämpfung und/oder zur präventiven Verhütung eines Befalls von Mikroorganismen und/oder zur Regulierung des Pflanzenwachstums.

11. Verfahren gemäss Anspruch 9, dadurch gekennzeichnet, dass es sich bei den Mikroorganismen um phytopathogene Pilze handelt.

12. Verfahren gemäss Anspruch 9 zur Wuchshemmung im Sinne einer Erhöhung der Knickfestigkeit und Halmverkürzung bei den Getreidesorten Hafer, Weizen, Gerste oder Roggen.

13. Verfahren gemäss Anspruch 9 zur Wuchshemmung bei Gräsern und Unkräutern.

14. Verfahren gemäss Anspruch 9 zur Wuchsregulierung bei Soja im Sinne einer Ertragssteigerung.

15. Verfahren gemäss Anspruch 9 zur Wuchshemmung von Bodenbedeckerpflanzen.

16. Verfahren gemäss Anspruch 9 zur Wuchshemmung und zur Wuchsregulierung von Baumwollpflanzen im Sinne einer Ertragssteigerung.

17. Verfahren gemäss Anspruch 9 zur Wuchsregulierung bei Getreidepflanzen im Sinne einer Ertragssteigerung.

18. Verfahren gemäss Anspruch 9 zur Seneszenzhemmung von Pflanzen im Sinne einer Ertragssteigerung.

**Claims** for the Contracting States: BE, CH, DE, FR, GB, IT, LI, NL, SE.

1. An azolylpropane derivative of the general formula I

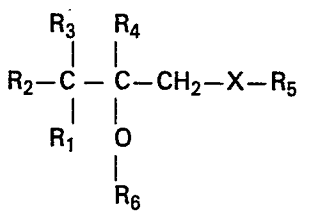

(I)

wherein

$R_1$ is 1H-1,2,4-triazol-1-yl or 1H-imidazol-1-yl, $R_2$ is $C_1$-$C_4$alkyl and $R_3$ is hydrogen, $C_1$-$C_4$alkyl, benzyl or benzyl monosubstituted by halogen, $R_4$ is $C_1$-$C_6$alkyl, phenyl or phenyl substituted by a member selected from the group consisting of halogen, cyano, nitro, $C_1$-$C_4$alkyl, $C_1$-$C_4$alkoxy, $C_1$-$C_4$alkylthio, $C_1$-$C_4$haloalkyl, $C_1$-$C_4$haloalkoxy or $C_1$-$C_4$haloalkylthio, $R_5$ is $C_1$-$C_8$alkyl, $C_3$-$C_8$cycloalkyl, phenylalkyl containing 1 to 4 carbon atoms in the alkyl moiety, or is phenyl or phenyl substituted by one or more member selected from the group consisting of halogen, cyano, nitro, $C_1$-$C_4$alkyl, $C_1$-$C_4$alkoxy, $C_1$-$C_4$alkylthio, $C_1$-$C_4$haloalkyl, $C_1$-$C_4$haloalkoxy or $C_1$-$C_4$haloalkylthio, $R_6$ is hydrogen, $C_1$-$C_4$alkyl, phenylalkyl containing 1 to 4 carbon atoms in the alkyl moiety, $C_1$-$C_6$alkylcarbonyl, carbamoyl carrying at the nitrogen atom, independently of one another, hydrogen, $C_1$-$C_4$alkoxy or $C_1$-$C_4$alkyl, or is $C_1$-$C_4$alkoxycarbonyl, $C_1$-$C_4$alkylsulfonyl, phenylsulfonyl which is unsubstituted or monosubstituted by $C_1$-$C_4$alkyl, or is sulfamoyl carrying at the nitrogen atom, independently of each other, hydrogen or $C_1$-$C_4$alkyl, X is oxygen or sulfur, or an acid addition salt, quaternary azolium salt or a metal complex thereof.

2. A compound according to claim 1, wherein $R_1$ is 1H-1,2,4-triazol-1-yl.

3. A compound according to claim 1, wherein $R_4$ is tert-butyl.

4. A compound according to claim 1, wherein $R_6$ is hydrogen, methyl, benzyl, acetyl, methylcarbamoyl, N,N-dimethylcarbamoyl, N-methoxy-N-methylcarbamoyl, methoxycarbonyl, ethoxycarbonyl, methylsulfonyl, 4-tolylsulfonyl, methylsulfamoyl or N,N-dimethylsulfamoyl.

5. A compound according to claim 1, wherein $R_1$ is 1H-1,2,4-triazol-1-yl, $R_2$ is $C_1$-$C_4$alkyl and $R_3$ is hydrogen or $C_1$-$C_4$alkyl, $R_4$ is $C_1$-$C_6$alkyl, phenyl or phenyl substituted by a member selected from the group consisting of halogen, cyano, nitro, $C_1$-$C_4$alkyl, $C_1$-$C_4$alkoxy, $C_1$-$C_4$alkylthio, $C_1$-$C_4$haloalkyl, $C_1$-$C_4$haloalkoxy or $C_1$-$C_4$haloalkylthio, $R_5$ is $C_1$-$C_8$alkyl, $C_3$-$C_8$cycloalkyl, phenylalkyl containing 1 to 4 carbon atoms in the alkyl moiety, or is phenyl or phenyl substituted by one or more members selected from the group consisting of halogen, cyano, nitro, $C_1$-$C_4$alkyl, $C_1$-$C_4$alkoxy, $C_1$-$C_4$alkylthio, $C_1$-$C_4$haloalkyl, $C_1$-$C_4$haloalkoxy or $C_1$-$C_4$haloalkylthio; and $R_6$ is hydrogen, methyl, benzyl, acetyl, methylcarbamoyl, N,N-dimethylcarbamoyl, N-methoxy-N-methylcarbamoyl, methoxycarbonyl, ethoxycarbonyl, methylsulfonyl, 4-tolylsulfonyl, methylsulfamoyl or N,N-dimethylsulfamoyl.

6. A compound according to claim 1, wherein $R_1$ is 1H-1,2,4-triazol-1-yl, $R_2$ is methyl or ethyl, $R_3$ is hydrogen, $R_4$ is isopropyl or tertbutyl, $R_5$ is phenyl or phenyl substituted by 1 or 2 methyl or nitro groups or 1 or 2 fluorine, chlorine or bromine atoms, X is oxygen and $R_6$ is hydrogen or methyl.

7. 1-(4-Chlorophenoxy)-2-tert-butyl-2-hydroxy-3-methyl-3-(1H-1,2,4 triazol-1-yl)propane according to claim 1.

8. 1-(4-Toluyloxy)-2-tert-butyl-2-hydroxy-3-methyl-3-(1H-1,2,4-triazol-1-yl)propane according to claim 1.

9. 1-(4-Chlorophenoxy)-2-tert-butyl-2-hydroxy-3-ethyl-3-(1H-1,2,4-triazol-1-yl)propane according to claim 1.

10. 1-(4-Fluorophenoxy)-2-tert-butyl-2-hydroxy-3-methyl-3-(1H-1,2,4-triazol-1-yl)propane according to claim 1.

11. 1-Phenoxy-2-tert-butyl-2-methoxy-3-methyl-3-(1H-1,2,4-triazol-1-yl)propane according to claim 1.

12. 1-(4-Fluorophenoxy)-2-tert-butyl-2-hydroxy-3-ethyl-3-(1H-1,2,4-triazol-1-yl)propane according to claim 1.

13. 1-(2-Fluorophenoxy)-2-tert-butyl-2-hydroxy-3-methyl-(1H-1,2,4-triazol-1-yl)propane according to claim 1.

14. 1-(4-Bromophenoxy)-2-tert-butyl-2-hydroxy-3-methyl-3-(1H-1,2,4 triazol-1-yl)propane according to claim 1.

15. 1-(4-Chloro-2-methylphenoxy)-2-tert-butyl-2-hydroxy-3-methyl-3-(1H-1,2,4-triazol-1-yl)propane according to claim 1.

16. 1-(4-Fluorophenoxy)-2-isopropyl-2-hydroxy-3-methyl-3-(1H-1,2,4-triazol-1-yl)propane according to claim 1.

17. An azolylmethyloxirane of the general formula II

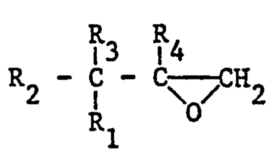

(II),

wherein $R_1$, $R_2$, $R_3$ and $R_4$ are as defined for formula I in claim 1.

18. A process for the preparation of a compound of the formula I, which comprises reacting an azolylmethyloxirane of the formula II

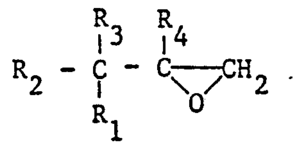 (II)

wherein $R_1$, $R_2$, $R_3$ and $R_4$ are as defined for formula I, with an alcohol or thioalcohol of the formula III

$$H-X-R_5 \qquad (III)$$

wherein X and $R_5$ are as defined for formula I, in the presence of a base and in an inert solvent, and, if desired, etherifying or esterifying the resultant compound of the formula Ia

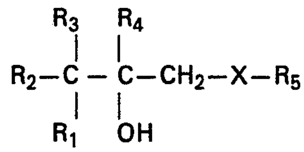 (Ia)

by reaction with an etherifying or esterifying agent of the formula IV

$$Y-R_6 \qquad (IV)$$

wherein $R_6$ is as defined for formula I and Y is a halogen atom or the radical of an organic or inorganic acid.

19. A composition for controlling or preventing attack by microorganisms and/or for regulating plant growth, which composition contains at least one compound of the formula I according to claim 1, together with carriers and/or other adjuvants.

20. A method of controlling phytopathogenic microorganisms or of protecting cultivated plants from attack by such microorganisms and/or of regulating plant growth, which method comprises applying an effective amount of a compound of the formula I as defined in claim 1 to said plants or to the locus thereof.

21. Use of a compound of formula I as claimed in claim 1 for controlling microorganisms and/or for preventing attack by microorganisms and/or for regulating plant growth.

22. A method according to claim 20, wherein the microorganisms to be controlled are phytopathogenic fungi.

23. A method of inhibiting plant growth according to claim 20, which comprises increasing the resistance to lodging and shortening the stalks of oats, wheat, barley or rye.

24. A method according to claim 20, which comprises inhibiting the growth of grasses and weeds.

25. A method of regulating plant growth according to claim 20, which comprises increasing the yield of soybeans.

26. A method of regulating plant growth according to claim 20, which comprises inhibiting the growth of cover plants.

27. A method of regulating plant growth according to claim 20, which comprises increasing the yield of cotton plants.

28. A method of regulating plant growth according to claim 20, which comprises increasing the yield of cereals.

29. A method according to claim 20, which comprises inhibiting the senescence of plants.

**Claims** for the Contracting State: AT.

1. A composition for controlling or preventing attack by microorganisms and/or for regulating plant growth, which composition contains, as active ingredient, at least one azolylpropane derivative of the general formula I

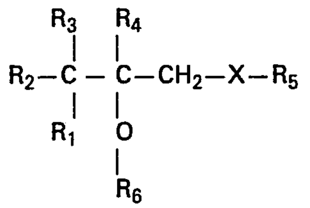

(I)

wherein

$R_1$ is 1H-1,2 4-triazol-1-yl or 1H-imidazol-1-yl, $R_2$ is $C_1$-$C_4$alkyl and $R_3$ is hydrogen, $C_1$-$C_4$alkyl, benzyl or benzyl monosubstituted by halogen, $R_4$ is $C_1$-$C_6$alkyl, phenyl or phenyl substituted by a member selected from the group consisting of halogen, cyano, nitro, $C_1$-$C_4$alkyl, $C_1$-$C_4$alkoxy, $C_1$-$C_4$alkylthio, $C_1$-$C_4$haloalkyl, $C_1$-$C_4$haloalkoxy or $C_1$-$C_4$haloalkylthio, $R_5$ is $C_1$-$C_8$alkyl, $C_3$-$C_8$cycloalkyl, phenylalkyl containing 1 to 4 carbon atoms in the alkyl moiety, or is phenyl or phenyl substituted by one or more member selected from the group consisting of halogen, cyano, nitro, $C_1$-$C_4$alkyl, $C_1$-$C_4$alkoxy, $C_1$-$C_4$alkylthio, $C_1$-$C_4$haloalkyl, $C_1$-$C_4$haloalkoxy or $C_1$-$C_4$haloalkylthio, $R_6$ is hydrogen, $C_1$-$C_4$-alkyl, phenylalkyl containing 1 to 4 carbon atoms in the alkyl moiety, $C_1$-$C_6$alkylcarbonyl, carbamoyl carrying at the nitrogen atom, independently of one another, hydrogen, $C_1$-$C_4$alkoxy or $C_1$-$C_4$alkyl, or is $C_1$-$C_4$alkoxycarbonyl, $C_1$-$C_4$alkylsulfonyl, phenylsulfonyl which is unsubstituted or monosubstituted by $C_1$-$C_4$alkyl, or is sulfamoyl carrying at the nitrogen atom, independently of each other, hydrogen or $C_1$-$C_4$alkyl, X is oxygen or sulfur, or an acid addition salt, quaternary azolium salt or a metal complex thereof, together with carriers and/or other adjuvants.

2. A composition according to claim 1, wherein $R_1$ is 1H-1,2,4-triazol-1-yl.

3. A composition according to claim 1, wherein $R_4$ is tert-butyl.

4. A composition according to claim 1, wherein $R_6$ is hydrogen, methyl, benzyl, acetyl, methylcarbamoyl, N,N-dimethylcarbamoyl, N-methoxy-N-methylcarbamoyl, methoxycarbonyl, ethoxycarbonyl, methylsulfonyl, 4-tolylsulfonyl, methylsulfamoyl or N,N-dimethylsulfamoyl.

5. A composition according to claim 1, wherein $R_1$ is 1H-1,2,4-triazol-1-yl, $R_2$ is $C_1$-$C_4$alkyl and $R_3$ is hydrogen or $C_1$-$C_4$alkyl, $R_4$ is $C_1$-$C_6$-alkyl, phenyl or phenyl substituted by a member selected from the group consisting of halogen, cyano, nitro, $C_1$-$C_4$alkyl, $C_1$-$C_4$alkoxy, $C_1$-$C_4$-alkylthio, $C_1$-$C_4$haloalkyl, $C_1$-$C_4$haloalkoxy or $C_1$-$C_4$haloalkylthio, $R_5$ is $C_1$-$C_8$alkyl, $C_3$-$C_8$cycloalkyl, phenylalkyl containing 1 to 4 carbon atoms in the alkyl moiety, or is phenyl or phenyl substituted by one or more members selected from the group consisting of halogen, cyano, nitro, $C_1$-$C_4$alkyl, $C_1$-$C_4$alkoxy, $C_1$-$C_4$alkylthio, $C_1$-$C_4$haloalkyl, $C_1$-$C_4$haloalkoxy or $C_1$-$C_4$haloalkylthio; and $R_6$ is hydrogen, methyl, benzyl, acetyl, methylcarbamoyl, N,N-dimethylcarbamoyl, N-methoxy-N-methylcarbanoyl, methoxycarbonyl, ethoxycarbonyl, methylsulfonyl, 4-tolylsulfonyl, methylsulfamoyl or N,N-dimethylsulfamoyl.

6. A composition according to claim 1, wherein $R_1$ is 1H-1,2,4-triazol-1-yl, $R_2$ is methyl or ethyl $R_3$ is hydrogen $R_4$ is isopropyl or tertbutyl, $R_5$ is phenyl or phenyl substituted by 1 or 2 methyl or nitro groups or 1 or 2 fluorine, chlorine or bromine atoms, X is oxygen and $R_6$ is hydrogen or methyl.

7. A composition as claimed in claim 1, which contains as active ingredient a compound selected from the group consisting of 1-(4-chlorophenoxy)-2-tert-butyl-2-hydroxy-3-methyl-3-(1H-1,2,4-triazol-1-yl)propane, 1-(4-toluyloxy)-2-tert-butyl-2-hydroxy-3-methyl-3-(1H-1,2,4-triazol-1-yl)propane, 1-(4-chlorophenoxy)-2-tert-butyl-2-hydroxy-3-ethyl-3-(1H-1,2,4-triazol-1-yl)propane, 1-(4-fluorophenoxy)-2-tert-butyl-2-hydroxy-3-methyl-3-(1H-1,2,4-triazol-1-yl)propane, 1-phenoxy-2-tert-butyl-2-methoxy-3-methyl-3-(1H-1,2,4-triazol-1-yl)propane, 1-(4-fluorophenoxy)-2-tert-butyl-2-hydroxy-3-ethyl-3-(1H-1,2,4-triazol-1-yl)propane, 1-(2-fluorophenoxy)-2-tert-butyl-2-hydroxy-3-methyl-(1H-1,2,4-triazol-1-yl)-propane, 1-(4-bromophenoxy)-2-tert-butyl-2-hydroxy-3-methyl-3-(1H-1,2,4-triazol-1-yl)propane, 1-(4-chloro-2-methylphenoxy)-2-tert-butyl-2-hydroxy-3-methyl-3-(1H-1,2,4-triazol-1-yl)propane, 1-(4-fluorophenoxy)-2-isopropyl-2-hydroxy-3-methyl-3-(1H-1,2,4-triazol-1-yl)propane.

8. A process for the preparation of a compund of the formula I, which comprises reacting an azolylmethyloxirane of the formula II

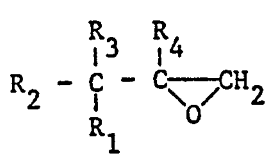

(II)

wherein $R_1$, $R_2$, $R_3$ and $R_4$ are as defined for formula I, with an alcohol or thioalcohol of the formula III

$$H—X—R_5 \qquad (III)$$

wherein X and $R_5$ are as defined for formula I, in the presence of a base and in an inert solvent, and, if desired, etherifying or esterifying the resultant compound of the formula Ia

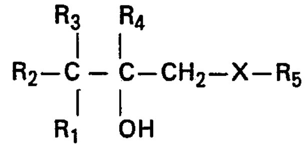

(Ia)

by reaction with an etherifying or esterifying agent of the formula IV

$Y-R_6$ (IV)

wherein $R_6$ is as defined for formula I and Y is a halogen atom or the radical of an organic or inorganic acid.

9. A method of controlling phytopathogenic microorganisms or of protecting cultivated plants from attack by such microorganisms and/or of regulating plant growth, which method comprises applying an effective amount of a compound of the formula I as defined in claim 1 to said plants or to the locus thereof.

10. Use of a compound of formula I as claimed in claim 1 for controlling microorganisms and/or for preventing attack by microorganisms and/or for regulating plant growth.

11. A method according to claim 9, wherein the microorganisms to be controlled are phytopathogenic fungi.

12. A method of inhibiting plant growth according to claim 9, which comprises increasing the resistance to lodging and shortening the stalks of oats, wheat, barley or rye.

13. A method according to claim 9 which comprises inhibiting the growth of grasses and weeds.

14. A method of regulating plant growth according to claim 9, which comprises increasing the yield of soybeans.

15. A method of regulating plant growth according to claim 9 which comprises inhibiting the growth of cover plants.

16. A method of regulating plant growth according to claim 9 which comprises increasing the yield of cotton plants.

17. A method of regulating plant growth according to claim 9 which comprises increasing the yield of cereals.

18. A method according to claim 9 which comprises inhibiting the senescence of plants.

**Revendications** pour les Etats contractants: BE, CH, DE, FR, GB, IT, LI, NL, SE.

1. Dérives de l'azolylpropane répondnant à la formule générale I

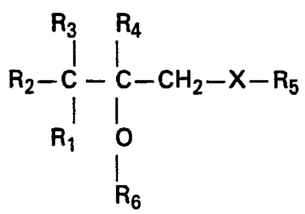

(I)

dans laquelle

$R_1$ représente un groupe 1H-1,2,4-triazole-1-yle ou 1H-imidazole-1-yle,

$R_2$ représente un groupe alkyle en C 1-C 4 et $R_3$ représente l'hydrogène, un groupe alkyle en C 1-C 4 ou un groupe benzyle éventuellement monosubstitué par un halogène,

$R_4$ représente un groupe alkyle en C 1-C 6 ou un groupe phényle éventuellement mono-substitué par un halogène, un groupe cyano, nitro, alkyle en C 1-C 4, alcoxy en C 1- C 4, alkylthio en C 1-C 4, halogénoalkyle en C 1-C 4, halogénoalcoxy en C 1-C 4 ou halogénoalkylthio en C 1-C 4,

$R_5$ représente un groupe alkyle en C 1-C 8, cycloalkyle en C 3-C 8, phénylalkyle contenant 1 à 4 atomes de carbone dans la partie alkyle ou phényle éventuellement mono- ou poly-substitué par des halogènes, des groupes cyano, nitro, alkyle en C 1-C 4, alcoxy en C 1-C 4, alkylthio en C 1-C 4, halogénoalkyle en C 1-C 4, halogénoalcoxy en C 1-C 4 ou halogénoalkylthio en C 1-C 4,

$R_6$ représente l'hydrogène, un groupe alkyle en C 1-C 4, phénylalkyle contenant 1 à 4 atomes de carbone dans la partie alkyle, (alkyle en C 1-C 6)-carbonyle, carbamoyle portant sur son atome d'azote, indépendamment les une des autres, l'hydrogène, des groupes alcoxy en C 1-C 4 ou alkyle en C 1-C 4, un groupe (alcoxy en C 1-C 4)-carbonyle, alkylsulfonyle en C 1-C 4, phénylsulfonyle éventuellement monosubstitué par un groupe alkyle en C 1-C 4 ou sulfamoyle portant sur son atome d'azote, indépendamment les uns des autres, l'hydrogène ou des groupes alkyle en C 1-C 4, et

X représente l'oxygène ou le soufre, y compris les sels formés par addition avec des acides, les sels d'azolium quaternaire et les complexes métalliques.

2. Composés selon la revendication 1, caractérisés en ce que $R_1$ représente un groupe 1H-1,2,4-triazole-1-yle.

3. Composés selon la revendication 1, caractérisés en ce que $R_4$ représente un groupe tert.-butyle.

4. Composés selon la revendication 1, caractérisés en ce que $R_6$ représente l'hydrogène, un groupe méthyle,

benzyle, acétyle, méthylcarbamoyle, N,N-diméthylcarbamoyle, N-méthoxy-N-méthylcarbamoyle, méthoxycarbonyle, éthoxycarbonyle, méthylsulfonyle, 4-tolylsulfonyle, méthylsulfamoyle ou N,N-diméthylsulfamoyle.

5. Composés selon la revendication 1, caractérisés en ce que $R_1$ représente un groupe 1H-1,2,4-triazole-1-yle, $R_2$ représente un groupe alkyle en C 1-C 4 et $R_3$ l'hydrogène ou un groupe alkyle en C 1-C 4, $R_4$ représente un groupe alkyle en C 1-C 6 ou un groupe phényle éventuellement monosubstitué par un halogène, un groupe cyano, nitro, alkyle en C 1-C 4, alcoxy en C 1-C 4, alkylthio en C 1-C 4, halogénoalkyle en C 1-C 4, halogénoalcoxy en C 1-C 4 ou halogénoalkylthio en C 1-C 4, $R_5$ représente un groupe alkyle en C 1-C 8, cycloalkyle en C 3-C 8, phénylalkyle contenant 1 à 4 atomes de carbone dans la partie alkyle ou phényle éventuellement mono- ou poly-substitué par des halogènes, des groupes cyano, nitro, alkyle en C 1-C 4, alcoxy en C 1-C 4, alkylthio en C 1-C 4, halogénoalkyle en C 1-C 4, halogénoalcoxy en C 1-C 4 ou halogénoalkylthio en C 1-C 4 et $R_6$ représente l'hydrogène, un groupe méthyle, benzyle, acétyle, méthylcarbamoyle, N,N-diméthylcarbamoyle, N-méthoxy-N-méthylcarbamoyle, méthoxycarbonyle, éthoxycarbonyle, méthylsulfonyle, 4-tolylsulfonyle, methylsulfamoyle ou N,N-diméthylsulfamoyle.

6. Composés selon la revendication 1, caractérisés en ce que $R_1$ représente un groupe 1H-1,2,4-triazole-1-yle, $R_2$ un groupe méthyle ou éthyle, $R_3$ l'hydrogène, $R_4$ un groupe isopropyle ou tert.-butyle, $R_5$ un groupe phényle non substitué ou mono- ou di-substitué par des groupes méthyle, nitro, le fluor, le chlore ou le brome, X représente l'oxygène et $R_6$ l'hydrogène ou un groupe méthyle.

7. Le 1-(4-chlorophénoxy)-2-tert.-butyl-2-hydroxy-3-méthyl-3-(1H-1,2,4-triazole-1-yl)-propane selon la revendication 1.

8. Le 1-(4-toluyloxy)-2-tert.-butyl-2-hydroxy-3-méthyl-3-(1H-1,2,4-triazole-1-yl)-propane selon la revendication 1.

9. Le 1-(4-chlorophénoxy)-2-tert.-butyl-2-hydroxy-3-éthyl-3-(1H-1,2,4-triazole-1-yl)-propane selon la revendication 1.

10. Le 1-(4-fluorophénoxy)-2-tert.-butyl-2-hydroxy-3-méthyl-3-(1H-1,2,4-triazole-1-yl)-propane selon la revendication 1.

11. Le 1-phénoxy-2-tert.-butyl-2-méthoxy-3-méthyl-3-(1H-1,2,4-triazole-1-yl)-propane selon la revendication 1.

12. Le 1-(4-fluorophénoxy)-2-tert.-butyl-2-hydroxy-3-éthyl-3-(1H-1,2,4-triazole-1-yl)-propane selon la revendication 1.

13. Le 1-(2-fluorophénoxy)-2-tert.-butyl-2-hydroxy-3-méthyl-(1H-1,2,4-triazole-1-yl)-propane selon la revendication 1.

14. Le 1-(4-bromophénoxy)-2-tert.-butyl-2-hydroxy-3-méthyl-3-(1H-1,2,4-triazole-1-yl)-propane selon la revendicstion 1.

15. Le 1-(4-chloro-2-méthylphénoxy)-2-tert.-butyl-2-hydroxy-3-méthyl-3-(1H-1,2,4-triazole-1-yl)-propane selon la revendication 1.

16. Le 1-(4-fluorophénoxy)-2-isopropyl-2-hydroxy-3-methyl-3-(1H-1,2,4-triazole-1-yl)-propane selon la revendication 1.

17. Azolylméthyloxirannes de formule générale II

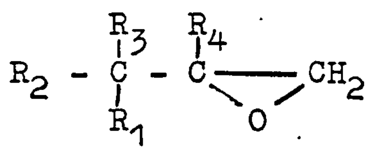

(II),

dans laquelle $R_1$, $R_2$, $R_3$ et $R_4$ ont les significations indiquées en référence à la formule I dans la revendication 1.

18. Procédé de préparation des composés de formule I, caractérisé en ce que l'on fait réagir un azolylméthyloxiranne de formule II

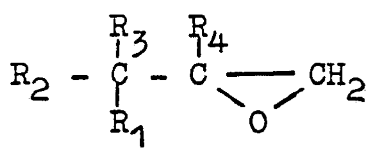

(II),

dans laquelle $R_1$, $R_2$, $R_3$ et $R_4$ ont les significations indiquées en référence à la formule I, en présence d'une base, dans un solvant inerte, avec un alcool ou thioalcool de formule III

$H-X-R_5$ (III)

dans laquelle X et $R_5$ ont les significations indiquées en référence à la formule I, ce qui donne un produit de formule Ia.

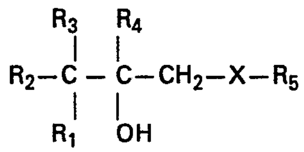

(Ia)

qu'on éthérifie ou qu'on estérifie si on le désire par réaction avec un agent éthérifiant ou estérifiant de formule IV

$Y-R_8$ (IV)

dans laquelle $R_6$ a les significations indiquées en référence à la formule I et Y représente un atome d'halogène ou un radical d'acide organique ou minéral.

19. Produit pour combattre ou prévenir une infestation par des microorganismes et/ou pour la régulation de la croissance des végétaux, caractérisé en ce qu'il contient en tant que substance active, avec des véhicules et/ou d'autres additifs, au moins un composé de formule I selon la revendication 1.

20. Procédé pour combattre ou prévenir une infestation des végétaux cultivés par des microorganismes phytopathogènes et/ou pour la régulation de la croissance des végétaux, caractérisé en ce que l'on applique sur les plantes ou leur habitat une quantité efficace d'un composé de formule I défini dans la revendication 1.

21. Utilisation des composés de formule I selon la revendication 1 pour combattre et/ou prévenir une infestation par des microorganismes et/ou pour la régulation de la croissance des vegetaux.

22. Procédé selon la revendication 20, caractérisé en ce que les microorganismes sont des mycètes phytopathogènes.

23. Procédé selon la revendication 20, pour l'inhibition de croissance dans le sens d'une amélioration de la résistance au pliage et d'un raccourcissement de la tige pour les céréales des espèces avoine, blé, orge ou seigle.

24. Procédé selon la revendication 20, pour l'inhibition de la croissance des graminées et des mauvaises herbes.

25. Procédé selon la revendication 20, pour la régulation de la croissance du soja dans le sens d'une augmentation de la récolte.

26. Procédé selon la revendication 20, pour l'inhibition de la croissance des végétaux de couvertures de sol.

27. Procédé selon la revendication 20, pour l'inhibition de la croissance et la régulation de la croissance des plants de coton dans le sens d'une augmentation de récolte.

28. Procédé selon la revendication 20, pour la régulation de la croissance des céréales dans le sens d'une augmentation de récolte.

29. Procédé selon la revendication 20, pour l'inhibition de la sénescence des plantes dans le sens d'une augmentation de récolte.

**Revendications** pour l'Etat contractant: AT.

1. Produit pour combattre ou prévenir une infestation par des microorganismes et/ou pour la régulation de la croissance des végétaux, caractérisé en ce qu'il contient en tant que substance active, avec des véhicules et/ou d'autres additifs, au moins un dérivé de l'azolylpropane répondant à la formule générale I

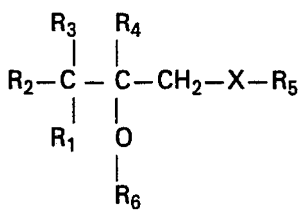

(I)

dans laquelle

$R_1$ représente un groupe 1H-1,2,4-triazole-1-yle ou 1H-imidazole-1-yle,

$R_2$ représente un groupe alkyle en C 1-C 4 et $R_3$ représente l'hydrogène, un groupe alkyle en C 1-C 4 ou benzyle éventuellement mono-substitué par un halogène,

$R_4$ représente un groupe alkyle en C 1-C 6 ou un groupe phényle éventuellement mono-substitué par un halogène, un groupe cyano, nitro, alkyle en C 1-C 4, alcoxy en C 1-C 4, alkylthio en C 1-C 4, halogénoalkyle en C

1-C 4, halogénoalcoxy en C 1-C 4 ou halogénoalkylthio en C 1-C 4,

$R_5$ représente un groupe alkyle en C 1-C 8, cycloalkyle en C 3-C 8, phénylalkyle contenant 1 à 4 atomes de carbone dans la partie alkyle ou phényle éventuellement mono- ou poly-substitué par des halogènes, des groupes cyano, nitro, alkyle en C 1-C 4, alcoxy en C 1-C 4, alkylthio en C 1-C 4, halogénoalkyle en C 1-C 4, halogénoalcoxy en C 1-C 4 ou halogénoalkylthio en C 1-C 4,

$R_6$ représente l'hydrogène, un groupe alkyle en C 1-C 4, phénylalkyle contenant 1 à 4 atomes de carbone dans, la partie alkyle, (alkyle en C 1-C 6)-carbonyle, carbamoyle portant, sur son atome d'azote, indépendamment les une des autres, l'hydrogène, des groupes alcoxy en C 1-C 4 ou alkyle en C 1-C 4, un groupe (alcoxy en C 1-C 4)-carbonyle, alkylsulfonyle en C 1-C 4, phénylsulfonyle éventuellement mono-substitué par un groupe alkyle en C 1-C 4 ou sulfamoyle portant sur son atome d'azote, indépendamment les une des autres, l'hydrogène ou des groupes alkyle en C 1-C 4, et

X représente l'oxygène ou le soufre, y compris les sels formés par addition avec des acides, sels d'azolium quaternaire et complexes métalliques.

2. Produit selon la revendiction 1, caractérisé en ce que $R_1$ représente un groupe 1H-1,2,4-triazole-1-yle.

3. Produit selon la revendication 1, caractérisé en ce que $R_4$ représente un groupe tert.-butyle.

4. Produit selon la revendication 1, caractérisé en ce que $R_6$ représente l'hydrogène, un groupe méthyle, benzyle, acétyle, méthylcarbamoyle, N,N-diméthylcarbamoyle, N-méthoxy-N-méthylcarbamoyle, méthoxycarbonyle, éthoxycarbonyle, méthylsulfonyle, 4-tolylsulfonyle, méthylsulfamoyle ou N,N-diméthylsulfamoyle.

5. Produit selon la revendication 1, caractérisé en ce que $R_1$ représente un groupe 1H-1,2,4-triazole-1-yle, $R_2$ un groupe alkyle en C 1-C 4 et $R_3$ l'hydrogène ou un groupe alkyle en C 1-C 4, $R_4$ un groupe alkyle en C 1-C 6 ou un groupe phényle éventuellement mono-substitué par un atome d'halogène, un groupe cyano, nitro, alkyle en C 1-C 4, alcoxy en C 1-C 4, alkylthio en C 1-C 4, halogénoalkyle en C 1-C 4, halogénoalcoxy en C 1-C 4 ou halogénoalkylthio en C 1-C 4, $R_5$ représente un groupe alkyle en C 1-C 8, cycloalkyle en C 3-C 8, phénylalkyle contenant 1 à 4 atomes de carbone dans la partie alkyle ou phényle éventuellement mono- ou poly-substitué par des halogènes, des groupes cyano, nitro, alkyle en C 1-C 4, alcoxy en C 1-C 4, alkylthio en C 1-C 4, halogénoalkyle en C 1-C 4, halogénoalcoxy en C 1-C 4 ou halogénoalkylthio en C 1-C 4 et $R_6$ représente l'hydrogène, un groupe méthyle, benzyle, acétyle, méthylcarbamoyle, N,N-diméthylcarbamoyle, N-méthoxy-N-méthylcarbamoyle, méthoxycarbonyle, éthoxycarbonyle, methylsulfonyle, 4-tolylsulfonyle, méthylsulfamoyle ou N,N-diméthylsulfamoyle.

6. Produit selon la revendication 1, caractérisé en ce que $R_1$ représente un groupe 1H-1,2,4-triazole-1-yle, $R_2$ un groupe méthyle ou éthyle, $R_3$ l'hydrogène, $R_4$ un groupe isopropyle ou tert.-butyle, $R_5$ un groupe phényle non substitué ou mono- ou di-substitué par des groupes méthyl, nitro, le fluor, le chlore ou le brome, X représente l'oxygène et $R_6$ l'hydrogène ou un groupe méthyle.

7. Produit selon la revendication 1, caractérisé en ce qu'il contient en tant que substance active un composé choisi dans le groupe formé par le 1-(4-chlorophénoxy)-2-tert.-butyl-2-hydroxy-3-méthyl-3-(1H-1,2,4-triazole-1-yl)-propane, le 1-(4-toluyloxy)-2-tert.-butyl-2-hydroxy-3-méthyl-3-(1H-1,2,4-triazole-1-yl)-propane, le 1-(4-chlorophénoxy)-2-tert.-butyl-2-hydroxy-3-éthyl-3-(1H-1,2,4-triazole-1-yl)-propane, le 1-(4-fluorophénoxy)-2-tert.-butyl-2-hydroxy-3-méthyl-3-(1H-1,2,4-triazole-1-yl)-propane, le 1-phénoxy-2-tert.-butyl-2-méthoxy-3-méthyl-3-(1H-1,2,4-triazole-1-yl)-propane, le 1-(4-fluorophénoxy)-2-tert.-butyl-2-hydroxy 3-éthyl-3-(1H-1,2,4-triazole-1-yl)-propane, le 1-(2-fluorophénoxy)-2-tert.-butyl-2-hydroxy-3-méthyl-(1H-1,2,4-triazole-1-yl)-propane, le 1-(4-bromophénoxy)-2-tert.-butyl-2-hydroxy-3-méthyl-3-(1H-1,2,4-triazole-1-yl)-propane, le 1-(4-chloro-2-méthylphénoxy)-2-tert.-butyl-2-hydroxy-3-méthyl-3-(1H-1,2,4-triazole-1-yl)-propane ou le 1-(4-fluorophénoxy)-2-isopropyl-2-hydroxy-3-méthyl-3-(1H-1,2,4-triazole-1-yl)-propane.

8. Procédé de préparation des composés de formule I, caractérisé en ce que l'on fait réagir un azolylméthyloxiranne de formule II

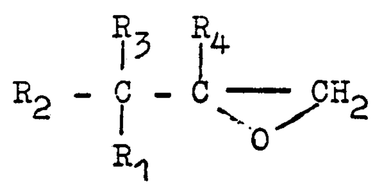

(II)

dans laquelle $R_1$, $R_2$, $R_3$ et $R_4$ ont les significations indiquées en référence à la formule I, en présence d'une base, dans un solvant inerte, avec un alcool ou thioalcool de formule III

$$H{-}X{-}R_5 \qquad (III)$$

dans laquelle X et $R_5$ ont les significations indiquées en référence à la formule I,
ce qui donne un produit de formule Ia

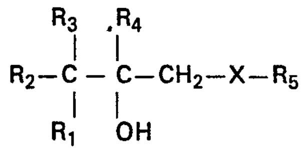

(Ia)

qu'on éthérifie ou qu'on estérifie si on le désire par réaction avec un agent étherifiant ou estérifiant de formule IV

$$Y{-}R_6 \qquad (IV)$$

dans laquelle $R_6$ a les significations indiquées en référence à la formule I, et Y représente un atome d'halogène ou un radical d'acide organique ou minéral.

9. Procédé pour combattre ou prévenir une infestation de végétaux cultivés par des microorganismes phytopathogènes et/ou pour la régulation de la croissance des végétaux, caractérisé en ce que l'on applique sur la plante ou son habitat une quantité efficace d'un composé de formule I défini dans la revendication 1.

10. Utilisation des composés de formule I selon la revendication 1 dans la lutte et/ou la prévention contre une infestation par des microorganismes et/ou pour la régulation de la croissance des végétaux.

11. Procédé selon la revendication 9, caractérisé en ce que les microorganismes sont des mycètes phytopathogènes.

12. Procédé selon la revendication 9, pour l'inhibition de la croissance, dans le sens d'une augmentation de la résistance au pliage et d'un raccourcissement de la tige, chez les céréales avoine, blé, orge ou seigle.

13. Procédé selon la revendication 9, pour l'inhibition de la croissance des graminées et des mauvaises herbes.

14. Procédé selon la revendication 9, pour la régulation de la croissance du soja dans le sens d'une augmentation de récolte.

15. Procédé selon la revendication 9, pour l'inhibition de la croissance des végétaux de couvertures de sol.

16. Procédé selon la revendication 9, pour l'inhibition de la croissance et la régulation de la croissance des plants de coton dans le sens d'une augmentation de récolte.

17. Procédé selon la revendication 9, pour la régulation de la croissance des céréales dans le sens d'une augmentation de récolte.

18. Procédé selon la revendication 9, pour l'inhibition de la sénescence des végétaux dans le sens d'une augmentation de récolte.